# EUROPEAN PATENT APPLICATION

(11) **EP 4 410 803 A1**
(43) Date of publication of application: **07.08.2024**
(21) Application number: 22876570.7
(22) Date of filing: 30.09.2022
(51) Int. Cl.: C07H 19/067

(54) **METHOD FOR PRODUCING 2' MODIFIED PYRIMIDINE NUCLEOSIDE**

(30) Priority: 30.09.2021 JP 2021161888
(71) Applicant: Nissan Chemical Corporation, Tokyo 103-6119 (JP)
(72) Inventor: FUKATSU, Yuichi, Funabashi-shi, Chiba 274-8507 (JP); MORODOME, Keisuke, Funabashi-shi, Chiba 274-8507 (JP)
(74) Representative: Hoffmann Eitle
(86) International application number: PCT/JP2022/036845
(87) International publication number: WO 2023/054708

(57) **Abstract**

[Problem to be Solved] To provide a novel method for producing a 2'-carbamoylalkyl-modified nucleoside having a pyrimidine base.

[Solution] A method for producing a 2'-carbamoylalkyl-modified uridine compound characterized by reacting an anhydrouridine compound and an amide compound having a protected or unprotected hydroxy group in a solvent such as an ether solvent in the presence of a boron reagent, and a method for producing a 2'-carbamoylalkyl-modified cytidine compound including a step of converting a uracil base of a 2'-carbamoylalkyl-modified uridine compound into a cytosine base.

## Description

### TECHNICAL FIELD

The present invention relates to a novel method for producing a 2'-modified pyrimidine nucleoside.

### BACKGROUND ART

A nucleic acid medicine is a pharmaceutical composed of a nucleic acid (oligonucleotide) that forms a complementary base pair with DNA or RNA that is a target, and is being expected as a new pharmaceutical. In addition, as nucleic acid units that are used in nucleic acid medicines, a variety of artificial nucleic acid units for which the structure of a natural nucleic acid is changed (artificial nucleosides or artificial nucleotides that are phosphate adducts of artificial nucleosides) have been developed. For example, methylated nucleosides, methoxyethylated nucleosides (MOE) or N-methylcarbamoyl ethylated nucleosides (MCE) of an oxygen atom at a sugar part 2'-position of a ribonucleoside have been reported (for example, refer to Patent Documents 1 and 2 and Non Patent Document 1). It has been reported that, among them, MCE oligonucleotides further improve the nuclease resistance than 2'-position methylated oligonucleotides (for example, refer to Non Patent Document 1).

It has been reported that 2'-(N-substituted carbamoyl) ethylated nucleosides, such as the MCE, can be produced by a Michael reaction of an acrylic acid ester and a ribonucleoside and subsequent amidation or the like (for example, refer to

### Patent Document 2 and Non Patent Document 1).

As a method for producing a nucleoside having a pyrimidine base such as thymine or uracil, a production method in which an anhydrouridine derivative and a protected or unprotected alcohol derivative corresponding to a substituent at the 2' position that is intended to be modified are reacted to substitute the 2' position has been reported (for example, refer to Patent Document 3 and Non Patent Documents 2 and 3).

### Related Art Documents

### Patent Documents

[Patent Document 1] JP 7-2889 A
[Patent Document 2] JP 5194256 B
[Patent Document 3] US 5739314 B

### Non Patent Document

[Non Patent Document 1] The Journal of Organic Chemistry, Vol. 76, p. 3042 (2011)
[Non Patent Document 2] Journal of Medicinal Chemistry, Vol. 51, p. 2766 (2008)
[Non Patent Document 3] Tetrahedron Letters, Vol. 48, p. 8554 (2007)

### SUMMARY OF THE INVENTION

### Problem to be Solved by the Invention

A method for producing a pyrimidine nucleoside having a N-methylcarbamoylmethyl group at the 2' position by the substitution of the 2' position is reported in Non Patent Document 2, but requires harsh conditions of a high temperature (150°C) in the absence of a solvent, with a low yield. A method for producing a pyrimidine nucleoside having a N-methylcarbamoylethyl group at the 2' position is reported in Patent Document 1, but includes complicated purification by column chromatography in all of Michael reaction of an acrylic acid ester, subsequent amidation reaction and deprotection reaction of a nucleic acid base between both reactions, making industrial production difficult.
An objective of the present invention is to provide a novel method for producing a 2'-carbamoylalkyl-modified nucleoside having a pyrimidine base.

### Means for Solving the Problem

As a result of intensive studies, the present inventors have found a novel method for producing a 2'-carbamoylalkyl-modified uridine compound characterized by reacting an anhydrouridine compound and an amide compound having a protected or unprotected hydroxy group in a solvent such as an ether solvent in the presence of a boron reagent.
Furthermore, the present inventors have found a novel method for producing a 2'-carbamoylalkyl-modified cytidine compound including a step of converting a uracil base of a 2'-carbamoylalkyl-modified uridine compound into a cytosine base, thereby completing the present invention. That is, the present invention relates to the followings.
[1] A method for producing a uridine compound of the following formula (II): 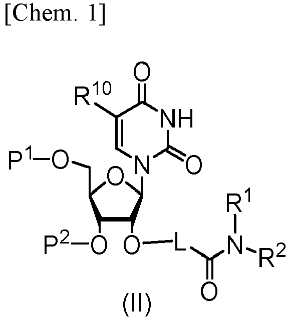 wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, and P¹ and P² are the same as or different from each other and are hydrogen atoms or protecting groups for a hydroxy group,
   the method comprising: a 2' substitution reaction step (ii) of reacting an anhydrouridine compound of the following formula (I) with an amide compound of the following formula (III) in a solvent in the presence of a boron reagent, 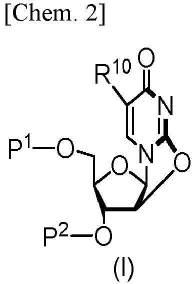
   wherein R¹⁰ is a hydrogen atom, a halogen atom, a C₁₋₆ alkyl group or a C₂₋₆ alkynyl group, and P¹ and P² are the same as or different from each other and are hydrogen atoms or protecting groups for a hydroxy group, 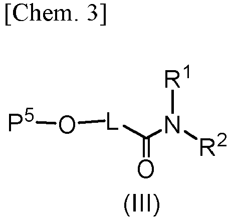
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, P⁵ is a hydrogen atom or a protecting group for a hydroxy group, and L is a substitutable C₁₋₆ alkylene group.
[2] The production method according to [1],
   wherein the boron reagent is a boron trifluoride diethyl ether complex.
[3] The production method according to [1] or [2],
   wherein 1 molar equivalent of the compound of formula (II) is reacted with the compound of formula (III) in the presence of 1.0 molar equivalent to 10 molar equivalents of the boron reagent.
[4] The production method according to any one of [1] to [3],
   wherein P⁵ is a silyl-based protecting group.
[5] The production method according to [4],
   wherein P⁵ is a trimethylsilyl group.
[6] The production method according to any one of [1] to [5],
   wherein L is a 1,2-ethylene group.
[7] The production method according to any one of [1] to [6],
   wherein R¹ and R² are the same as or different from each other and are hydrogen atoms or C₁₋₆ alkyl groups.
[8] The production method according to [7],
   wherein R¹ is a hydrogen atom and R² is a C₁₋₃ alkyl group.
[9] The production method according to any one of [1] to [8],
   wherein R¹⁰ is a hydrogen atom or a methyl group.
[10] The production method according to any one of [1] to [9],
   wherein the solvent is selected from ether solvents or amide solvents.
[11] The production method according to any one of [1] to [10],
   wherein P¹ is a silyl-based protecting group and P² is a hydrogen atom.
[12] The production method according to [11],
   wherein P¹ is a t-butyldiphenylsilyl group.
[13] The production method according to [11] or [12],
   wherein the solvent is selected from ether solvents.
[14] The production method according to [13],
   wherein the solvent is at least one selected from the group consisting of 1,2-dimethoxyethane, tetrahydrofuran, 4-methyltetrahydropyran and dioxane.
[15] The production method according to any one of [11] to [14], further comprising a step (iii) of deprotecting the silyl-based protecting group of P¹ to generate a 5'-hydroxy group, after the 2' substitution reaction step (ii).
[16] The production method according to any one of [1] to [10],
   wherein P¹ and P² are hydrogen atoms.
[17] The production method according to [16],
   wherein the solvent is selected from amide solvents.
[18] The production method according to [17],
   wherein the solvent is N,N-dimethylacetamide.
[19] A method for producing a uridine amidite compound of the following formula (V), the method comprising:
   producing a compound of the following formula (II-2) by using the production method according to any one of [1] to [18]; 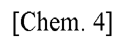 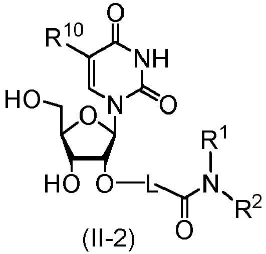
   wherein R¹, R², R¹⁰ and L are as described above, and
   then a step (iv) of converting a 5'-hydroxy group of the compound of formula (II-2) into a protecting group for a hydroxy group; and a step (v) of converting a 3'-hydroxy group into an amidite group of the following formula (PA) with an amidite-forming reagent, 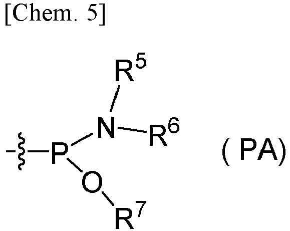
   wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group, 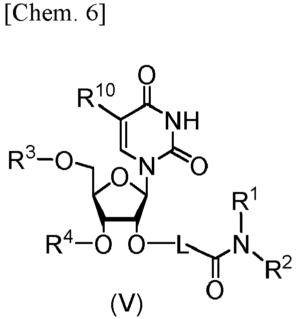
   wherein R¹, R², R¹⁰ and L are as described above, R³ is a protecting group for a hydroxy group, and R⁴ is an amidite group of formula (PA).
[20] A method for producing a cytosine compound of the following formula (B), the method comprising:
   a step (b) of reacting, in a solvent,
   a protected uridine compound of the following formula (A) with a sulfonyl halide compound, and then with ammonia water, 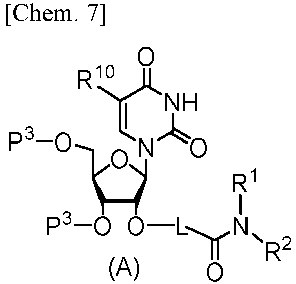
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, and P³ are the same as or different from each other and are substitutable benzoyl groups, 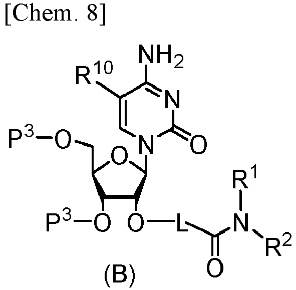
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, and P³ are the same as or different from each other and are substitutable benzoyl groups.
[21] The production method according to [20],
   wherein the sulfonyl halide compound is of the following formula (C): 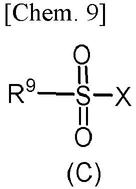
   wherein X is a halogen atom, and R⁹ is a substitutable C₁₋₆ alkyl group or a substitutable C₆₋₁₄ aryl group.
[22] The production method according to [21],
   wherein the sulfonyl halide compound is at least one selected from the group consisting of methanesulfonyl chloride, p-toluenesulfonyl chloride, and 2,4,6-triisopropylbenzenesulfonyl chloride.
[23] The production method according to any one of [20] to [22], comprising, before the step (b),
   a step (a1) of reacting a uridine compound of the following formula (II-2) with a benzoylation reagent to synthesize the protected uridine compound of formula (A), and a step (a2) of obtaining the protected uridine compound of formula (A) by solid-liquid separation, 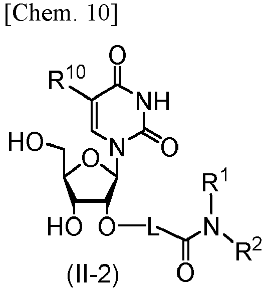
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, and Lisa substitutable C₁₋₆ alkylene group.
[24] A method for producing a cytosine amidite compound of the following formula (G), comprising
   producing a cytosine compound of formula (B) by the production method according to any one of [20] to [23], and using the cytosine compound as a starting material, 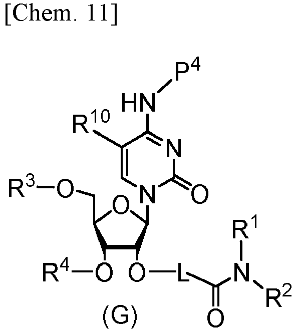
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, P⁴ is a protecting group for an amino group, R³ is a protecting group for a hydroxy group, R⁴ is a cytosine amidite compound of the following formula (PA), wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group, 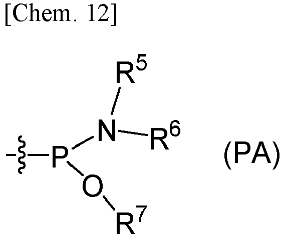
[25] The production method according to [24], comprising
   a step (c) of reacting the cytosine compound of formula (B) with a primary or secondary alkylamine to obtain a cytosine compound of the following formula (D): 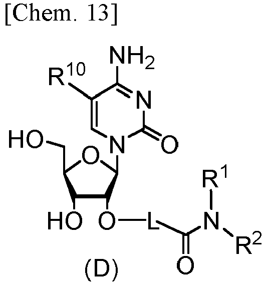
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, and Lisa substitutable C₁₋₆ alkylene group.
[26] The production method according to [25], further comprising:
   a step (d1) of converting the 5'-hydroxy group in the cytosine compound of formula (D) into a protecting group for a hydroxy group; and
   a step (d2) of protecting an amino group of a cytosine base to produce a cytosine compound of the following formula (F): 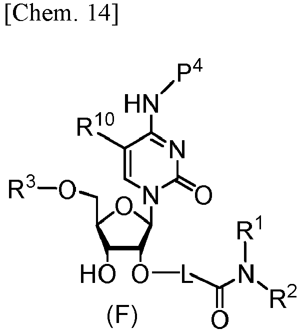
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, P⁴ is a protecting group for an amino group, and R³ is a protecting group for a hydroxy group.
[27] The production method according to [26], further comprising
   a step (e) of converting the cytosine compound of formula (F) into a cytosine amidite compound of the following formula (G) with an amidite-forming reagent, 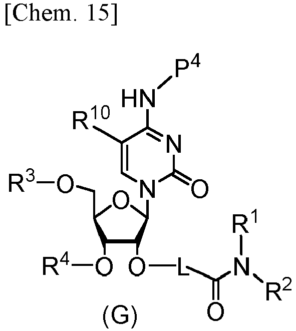
   wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, P⁴ is a protecting group for an amino group, R³ is a protecting group for a hydroxy group, R⁴ is a cytosine amidite compound of the following formula (PA), wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group, 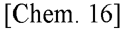 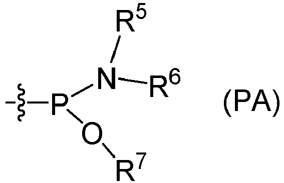
[28] The production method according to [27],
   wherein R⁵ and R⁶ are i-propyl groups, and R⁷ is a 2-cyanoethyl group.
[29] The production method according to any one of [24] to [28],
   wherein R³ is a 4,4'-dimethoxytrityl group.
[30] The production method according to any one of [24] to [29],
   wherein P⁴ is an acetyl group, a propionyl group, an i-butyryl group, a pivaloyl group, a n-butyryl group, a benzoyl group or a phenoxyacetyl group.

### Effects of the Invention

The present invention provides novel methods for producing 2'-carbamoylalkyl-modified uridine compound and a 2'-carbamoylalkyl-modified cytidine compound. According to the present invention, it is possible to cause a 2'-carbamoylalkyl modification step to progress with a high yield under a mild condition with no need of a high temperature. In addition, the present invention makes it possible to reduce purification by complicated column chromatography. As described above, the present invention makes it possible to provide a novel production method suitable for the industrial production of a 2'-carbamoylalkyl-modified pyrimidine nucleoside.

### MODES FOR CARRYING OUT THE INVENTION

Hereinafter, the present invention will be described in more detail. In the present invention, "n-" means normal, "i-" means iso, "s-" mean secondary, "t-" and "tert-" mean tertiary, "o-" means ortho, "m-" means metha, "p-" means para, "Me" means methyl, "TMS" means trimethylsilyl, "DMTr" means dimethoxytrityl, "TBDPS" means tert-butyldiphenylsilyl, "Bz" means benzoyl, and "Ac" means acetyl.

First, terms that are used to describe chemical structures in the present specification will be described.

"C₁₋₃ alkyl group" means a linear or branched alkyl group having 1 to 3 carbon atoms, and specific examples thereof include methyl group, ethyl group, n-propyl group and i-propyl group. "C₁₋₆ alkyl group" means a linear or branched alkyl group having 1 to 6 carbon atoms, and specific examples thereof include methyl group, ethyl group, n-propyl group, i-propyl group, n-butyl group, i-butyl group, s-butyl group, t-butyl group, n-pentyl group, 2-methylbutyl group, 3-methylbutyl group, n-hexyl group, 2-methylpentyl group, 3-methylpentyl group, 4-methylpentyl group, 2-ethylbutyl group and 3-ethylbutyl group.

"C₃₋₆ cycloalkyl group" means a cyclic alkyl group having 3 to 6 carbon atoms as atoms configuring a ring, and specific examples thereof include cyclopropyl group, cyclobutyl group, cyclopentyl group and cyclohexyl group.

"C₇₋₁₆ aralkyl group" means an alkyl group having an aromatic hydrocarbon as a substituent and having 7 to 16 carbon atoms in the entire substituent. Specific examples thereof include phenylmethyl group (benzyl group), 1-phenylethyl group, 2-phenylethyl group, 1-phenylpropyl group, 2-phenylpropyl group, 3-phenylpropyl group, naphthalen-1-ylmethyl group, naphthalen-2-ylmethyl group, naphthalen-1-ylethyl group, naphthalen-2-ylethyl group, anthracen-1-ylmethyl group, anthracen-2-ylmethyl group and anthracen-9-ylmethyl group.

"C₂₋₆ alkenyl group" means a linear or branched alkenyl group having 2 to 6 carbon atoms and having at least one double bond, and specific examples thereof include ethenyl group, 1-propenyl group, 2-propenyl group, 1-butenyl group, 2-butenyl group, 3-butenyl group, 1-pentenyl group, 2-pentenyl group, 3-pentenyl group and 4-pentenyl group.

"C₂₋₆ alkynyl group" means a linear or branched alkynyl group having 2 to 6 carbon atoms and having at least one triple bond, and specific examples thereof include ethynyl group, 1-propynyl group, 2-propynyl group, 1-butynyl group, 2-butynyl group, 3-butynyl group, 1-pentynyl group, 2-pentynyl group, 3-pentynyl group and 4-pentynyl group.

"C₁₋₆ alkylene group" means a linear or branched alkylene group having 1 to 6 carbon atoms, and specific examples thereof include methylene group, 1,1-ethylene group, 1,2-ethylene group, trimethylene group, tetramethylene group, pentamethylene group, hexamethylene group, 1-methyltrimethylene group, 2-methyltrimethylene group, 1,1-dimethylethylene group, 1-methyltetramethylene group, 2-methyltetramethylene group, 1,1-dimethyltrimethylene group, 1,2-dimethyltrimethylene group, 2,2-dimethyltrimethylene group, 1-ethyltrimethylene group and the like. "C₂₋₆ alkylene group" means a linear or branched alkynylene group having 2 to 6 carbon atoms in which two different carbon atoms have a valency of radical, and specific examples thereof include 1,2-ethylene group, trimethylene group, tetramethylene group, pentamethylene group, hexamethylene group, 1-methyltrimethylene group, 2-methyltrimethylene group, 1,1-dimethylethylene group, 1-methyltetramethylene group, 2-methyltetramethylene group, 1,1-dimethyltrimethylene group, 1,2-dimethyltrimethylene group, 2,2-dimethyltrimethylene group, 1-ethyltrimethylene group and the like.

"C₁₋₆ alkoxy group" means the "C₁₋₆ alkyl group" to which an oxy group (-O-) is bonded, and specific examples thereof include methoxy group, ethoxy group, n-propoxy group, i-propoxy group, n-butoxy group, i-butoxy group, s-butoxy group, t-butoxy group, n-pentyloxy group, 1-methyl-n-butoxy group, 2-methyl-n-butoxy group, 3-methyl-n-butoxy group, 1,1-dimethyl-n-propoxy group, 1,2-dimethyl-n-propoxy group, 2,2-dimethyl-n-propoxy group, 1-ethyl-n-propoxy group, n-hexyloxy group, 1-methyl-n-pentyloxy group, 2-methyl-n-pentyloxy group, 3-methyl-n-pentyloxy group, 4-methyl-n-pentyloxy group, 1,1-dimethyl-n-butoxy group, 1,2-dimethyl-n-butoxy group, 1,3-dimethyl-n-butoxy group, 2,2-dimethyl-n-butoxy group, 2,3-dimethyl-n-butoxy group, 3,3-dimethyl-n-butoxy group, 1-ethyl-n-butoxy group, 2-ethyl-n-butoxy group, 1,1,2-trimethyl-n-propoxy group, 1,2,2-trimethyl-n-propoxy group, 1-ethyl-1-methyl-n-propoxy group, 1-ethyl-2-methyl-n-propoxy group and the like.

"C₆₋₁₄ aryl group" means an aromatic hydrocarbon group having 6 to 14 carbon atoms, and specific examples thereof include phenyl group, 1-naphthyl group, 2-naphthyl group, 1-anthracenyl group, 2-anthracenyl group and 9-anthracenyl group.

"Substitutable" means unsubstituted or substituted with any number of any substituents. "Substituted" means substituted with any number of any substituents. Any number is, for example, one to three. The above-mentioned "any substituent" is not particularly limited as long as a substituent does not adversely affect the present reaction. Examples of any substituent include a halogen atom, hydroxy group, a protective form of hydroxy group, amino group, a protective form of amino group, carboxy group, a protective form of carboxy group, a nitro group, a cyano group, C₁₋₆ alkyl group, C₁₋₆ alkoxy group, C₃₋₆ cycloalkyl group, C₂₋₆ alkenyl group, C₆₋₁₄ aryl group, C₇₋₁₆ aralkyl group, five to ten-membered heterocyclic group and the like and further include phenyl group substituted with the above-listed substituent.

"To be bonded to each other to form a ring together with the nitrogen atom to which they are bonded" means that two substituents bonded to one nitrogen atom are bonded to each other to form a five to seven-membered ring together with the nitrogen atom. Specific examples of the five to seven-membered ring include pyrrolidine, piperidine, azepine, morpholine and homomorpholine.

A halogen atom means a fluorine atom, a chlorine atom, a bromine atom or an iodine atom.

"Protecting group for hydroxy group" refers to a protecting group that is normally used in organic synthetic chemistry (particularly, nucleic acid synthesis). Examples thereof include C₁₋₆ alkyl group (methyl group, ethyl group, tert-butyl group or the like), allyl group, benzyl-based protecting group (benzyl group, p-methoxybenzyl group or the like), acyl-based protecting group (formyl group, acetyl group, benzoyl group or the like), acetal-based protecting group (methoxymethyl group, ethoxymethyl group, 2-tetrahydropyranyl group or the like), silyl-based protecting group (trimethylsilyl group, triisopropylsilyl group, t-butyldimethylsilyl group, t-butyldiphenylsilyl group or the like), triarylmethyl-based protecting group (trityl group, 4,4'-dimethoxytrityl group or the like), but are not limited thereto. A protecting group for a hydroxy group that is particularly preferable in each step will be described below.

"Protecting group for amino group" refers to a protecting group that is normally used in organic synthetic chemistry (particularly, nucleic acid synthesis). Examples thereof include acyl-based protecting group (formyl group, acetyl group, benzoyl group, isobutyryl group, pivaloyl group, trifluoroacetyl group, phenoxyacetyl group or the like), carbamate-based protecting group (tert-butoxycarbonyl group, benzyloxycarbonyl group, 9-fluorenylmethyloxycarbonyl group or the like), sulfone-based protecting group (mesyl group, p-toluenesulfonyl group, 2-nitrobenzenesulfonyl group or the like), triarylmethyl-based protecting group (trityl group, 4,4'-dimethoxytrityl group or the like), amidinyl-based protecting group (N,N-dimethylaminomethylene group, N,N-diethylaminomethylene group, N,N-diisopropylaminomethylene group, 1-pyrrolidinomethylene group or the like), but are not limited thereto. A protecting group for an amino group that is particularly preferable in each step will be described below.

"Protecting group for phosphate group" refers to a protecting group that is normally used in organic synthetic chemistry (particularly, nucleic acid synthesis). Examples thereof include alkyl-based protecting group (2-cyanoethyl group, 2,2,2-trichloroethyl group or benzyl group), aryl-based protecting group (2-chlorophenyl group or 4-chlorophenyl group) and the like, but are not limited thereto.

"Deprotection" means deprotecting a protecting group that is used in normal organic synthetic reactions. From a protected hydroxy group, a protected amino group, and a protected phosphate group, a protecting group can be deprotected by performing a deprotection reaction (for example, refer to Protective Groups in Organic Synthesis, Fourth edition, written by T. W. Greene, John Wiley & Sons Inc. (2006) or the like).

The kind of a base that is used in the present reaction is not limited as long as the base does not affect the reaction, and examples thereof include trialkylamines (triethylamine, N,N-diisopropylethylamine and the like), N-methylmorpholine, pyridines, 1,8-diazabicyclo[5.4.0]-7-undecene, sodium carbonate, potassium carbonate, cesium carbonate, sodium hydroxide, potassium hydroxide, cesium hydroxide, sodium methoxide, potassium methoxide, sodium tert-butoxide, potassium tert-butoxide and the like. A base that is particularly preferable in each step will be described below.

One base may be used singly or two or more bases may be used in combination.

A solvent that is used in the present invention is not limited as long as the solvent does not impair the progress of a reaction, and preferable examples thereof include an aprotic polar organic solvent (for example, an amide-based solvent such as N,N-dimethylformamide or N,N-dimethylacetamide, a nitrile-based solvent such as acetonitrile, dimethyl sulfoxide, tetramethylurea, sulfolane, N-methyl-2-pyrrolidone, N,N-dimethylimidazolidinone or the like), an ether-based solvent (for example, diethyl ether, diisopropyl ether, t-butyl methyl ether, tetrahydrofuran, dioxane or the like), an aliphatic hydrocarbon-based solvent (for example, pentane, n-hexane, c-hexane, octane, decane, decalin, petroleum ether or the like), an aromatic hydrocarbon-based solvent (benzene, nitrobenzene, toluene, xylene, mesitylene, tetralin or the like), a pyridine-based solvent, a halogenated hydrocarbon-based solvent (for example, chloroform, dichloromethane, dichloroethane, carbon tetrachloride or the like), a halogenated aromatic hydrocarbon-based solvent (chlorobenzene, o-dichlorobenzene or the like), a lower aliphatic acid ester-based solvent (for example, methyl acetate, ethyl acetate, butyl acetate, methyl propionate or the like), an alkoxyalkane-based solvent (for example, dimethoxyethane, diethoxyethane or the like), an alcoholic-based solvent (for example, methanol, ethanol, n-propanol, i-propanol, n-butanol, tert-butanol or the like), acetic acid and water. A solvent that is particularly preferable in each step will be described below.

One solvent may be used singly or a solvent mixture of two or more solvents may be used.

"Solid-liquid separation" refers to an operation of separating a solid that disperses in a liquid from the liquid. Specific examples of the operation include filtration, centrifugation and the like, and filtration is preferable.

"Crystal" refers to a solid in which atoms or molecules are regularly arranged. For example, the crystal can be confirmed by detecting a characteristic peak with a powder X-ray diffractometer. In the powder X-ray diffractometer, a characteristic peak can be detected with an error of approximately ±0.2°.

Hereinafter, the present invention will be described in detail. In drawings shown below until examples, unless particularly otherwise described, R¹ to R⁷, R⁹ to R¹⁰, R¹¹, P¹ to P⁵, L and X in formulae are the same meanings as described above.

A method for producing a series of compounds of the present invention will be shown in the following schemes. In the schemes, "step" means steps. In detailed description to be described below, "compound of formula (formula number)" will be referred to as "formula (formula number) compound" or "compound (formula number) in some cases. For example, a compound of formula (I) will be referred to as formula (I) compound or compound (I) and a compound of formula (A) will be referred to as formula (A) compound or compound (A) in some cases.

### [Method for producing 2'-carbamoylalkyl-modified uridine compound]

The present invention relates to a method for producing a uridine compound of the following formula (II), and the present production method includes a step (2' substitution reaction step) (ii) of reacting an anhydrouridine compound of formula (I) with an amide compound of formula (III) in a solvent in the presence of a boron reagent to substitute a 2' position as shown in the following scheme. The present step makes it possible to synthesize a desired 2' modified pyrimidine nucleoside.

### Step (ii)

### (Scheme I)

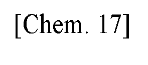

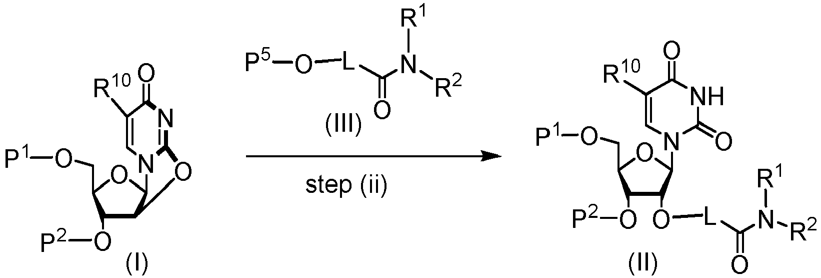

R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups. R¹ and R² are the same as or different from each other and are preferably hydrogen atoms or C₁₋₆ alkyl groups. At least one of R¹ and R² is preferably a hydrogen atom, and it is more preferable that R¹ is a hydrogen atom and R² is a C₁₋₆ alkyl group. It is still more preferable that R¹ is a hydrogen atom and R² is a C₁₋₃ alkyl group. It is far still more preferable that R¹ is a hydrogen atom and R² is a methyl group. In addition, it is also preferable that both R¹ and R² are methyl groups.

R¹⁰ is a hydrogen atom, a halogen atom, a C₁₋₆ alkyl group or a C₂₋₆ alkynyl group.
R¹⁰ is preferably a hydrogen atom or a C₁₋₆ alkyl group, more preferably a hydrogen atom or a C₁₋₃ alkyl group, still more preferably a hydrogen atom or a methyl group and particularly preferably a methyl group.

L is a substitutable C₁₋₆ alkylene group, preferably a C₁₋₆ alkylene group, more preferably a C₂₋₆ alkylene group and particularly preferably a 1,2-ethylene group.

P¹ and P² are the same as or different from each other and are hydrogen atoms or protecting groups for a hydroxy group.

Here, the protecting group for a hydroxy group may be, as described above, a protecting group that is normally used in organic synthetic chemistry (particularly, nucleic acid synthesis).
P¹ and P² may be each independently a group that protects a 5'-hydroxy group and a 3'-hydroxy group, and P¹ and P² may be groups that are bonded to each other to protect a 3'-hydroxy group and a 5'-hydroxy group as shown in formula (Sx). 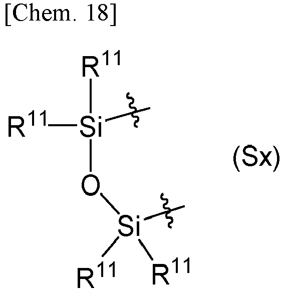
wherein R¹¹ are the same as or different from each other and are substitutable C₁₋₆ alkyl groups or substitutable phenyl groups.
R¹¹ are the same as or different from each other and are preferably C₁₋₆ alkyl groups and more preferably isopropyl groups. A compound having the protecting group shown by formula (Sx) can be synthesized by a method described in Non Patent Document 1 or a method according thereto.

P¹ is preferably a silyl-based protecting group, more preferably a triisopropylsilyl group, a t-butyldimethylsilyl group or a t-butyldiphenylsilyl group, still more preferably a t-butyldimethylsilyl group or a t-butyldiphenylsilyl group and particularly preferably a t-butyldiphenylsilyl group.
P¹ may be a hydrogen atom.

P² is preferably a hydrogen atom or a silyl-based protecting group and particularly preferably a hydrogen atom.

P⁵ is a hydrogen atom or a protecting group for a hydroxy group. The protecting group may be, as described above, a protecting group that is normally used in organic synthetic chemistry (particularly, nucleic acid synthesis).
P⁵ is preferably a protecting group for a hydroxy group. P⁵ is more preferably a silyl-based protecting group, still more preferably a trimethylsilyl group, a triethylsilyl group or a triisopropylsilyl group and particularly preferably a trimethylsilyl group.

An amide compound of formula (III-1) that is the amide compound of formula (III) in which P⁵ is a hydrogen atom can be produced by reacting a corresponding lactone compound and an amine compound (HNR₁(R₂)) in a solvent such as an alcoholic solvent. A base may be used in the reaction. An amide compound of formula (III-2) in which P⁵ is a protecting group (P^{5PR}) of a hydroxy group can be produced by protecting a hydroxy group (-OH) in the amide compound in which P⁵ is a hydrogen atom with a protecting group for a hydroxy group.

### (Scheme II)

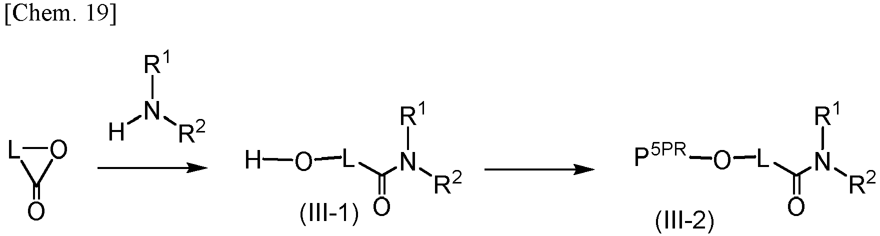

In the scheme, R¹, R² and L are the same as described above, and P^{5PR} is a protecting group for a hydroxy group.
For the protection of the hydroxy group, a protecting group that is often used in organic synthetic chemistry can be used, and the hydroxy group can be protected by, for example, a method described in Protective Groups in Organic Synthesis, Fourth edition, written by T. W. Greene, John Wiley & Sons Inc. (2006).

As the boron reagent that is used in the present step, a borane trifluoride diethyl ether complex, a borane tetrahydrofuran complex, a borane dimethyl sulfide complex or a pinacolborane can be used. A borane trifluoride diethyl ether complex is preferable.

For example, in a case where a protected alcohol amide compound (P⁵ is a protecting group for a hydroxy group, compound (III-2)) is used as the amide compound of formula (III), the boron reagent is preferably a boron trifluoride diethyl ether complex. In addition, in a case where an alcohol amide compound (P⁵ is a hydrogen atom, compound (III-1)) is used as the amide compound of formula (III), the boron reagent is preferably a borane tetrahydrofuran complex.

The amount of the boron reagent used is preferably 1.0 to 10 molar equivalents, more preferably 1.0 to 5.0 molar equivalents and still more preferably 1.5 to 4.0 molar equivalents, with respect to 1 molar equivalent of the compound (I).

The amount of the amide compound of formula (III) used is preferably 2.0 to 10.0 molar equivalents, more preferably 2.0 to 7.0 molar equivalents and still more preferably 3.0 to 6.0 molar equivalents, with respect to 1 molar equivalent of the compound (I).

The solvent that is used in the present step may be a solvent in which the reaction favorably progresses, and examples thereof include alcoholic-based solvents, ether-based solvents, amide-based solvents and the like. An ester-based solvent or an amide-based solvent is preferably used.

For example, in a case where a protected alcohol amide compound (compound (III-2)) is used as the amide compound of formula (III), the solvent is preferably an ether-based solvent, more preferably dioxane, diethyl ether, tetrahydrofuran, 4-methyltetrahydropyran, 1,2-dimethoxyethane or diethylene glycol dimethyl ether, still more preferably dioxane, 1,2-dimethoxyethane, diethyl ether, tetrahydrofuran or 4-methyltetrahydropyran, far still more preferably dioxane, 1,2-dimethoxyethane or 4-methyltetrahydropyran and particularly preferably dioxane. In a case where an alcohol amide compound (compound (III-1)) is used as the amide compound of formula (III), the solvent is preferably an amide-based solvent.

In addition, when P¹ is a silyl-based protecting group and P² is a hydrogen atom, the solvent is preferably an ether-based solvent and the most preferably 1,2-dimethoxyethane, tetrahydrofuran, 4-methyltetrahydropyran or dioxane. In addition, when P¹ and P² are hydrogen atoms, the solvent is preferably an amide-based solvent and the most preferably N,N-dimethylacetamide.

The reaction temperature of the present step is preferably 20 to 150°C, more preferably 30 to 120°C, still more preferably 40 to 110°C, far still more preferably 60 to 100°C, even far still more preferably 80 to 100°C and particularly preferably 80 to 95°C.

The reaction time of the present step is, for example, 0.5 to 48 hours, preferably 1 to 24 hours, more preferably 2 to 8 hours and particularly preferably 3 to 6 hours.

In the formula (I) compound, in a case where the 5' position (P¹) is a protecting group for a hydroxy group (for example, a silyl-based protecting group), the above-described 2' substitution reaction step quantitatively progresses, and it is possible to perform the present step at a lower temperature (for example, 120°C or lower, 110°C or lower, 100°C or lower or 95°C or lower). Furthermore, in this case, the formula (II) compound can be obtained by a separation operation from a crude product obtained after the 2' substitution reaction step, which is preferable.

In a case where P¹, P² or both P¹ and P² are protecting groups for a hydroxy group in the compound of formula (II), these can be deprotected to obtain a compound having hydroxy groups at the 3' position and the 5' position. The present compound serves as a starting material for the production of a uridine amidite compound of formula (V), which will be described below, and serves as a starting material in a step (a1), which will be described below.

A compound that is the formula (I) compound in which P¹ is a protecting group for a hydroxy group can be obtained by protecting the 5'-hydroxy group in the following compound (I-1) that is the formula (I) compound in which P¹ and P² are hydroxy groups.
In the following compound (I-2) in which P¹ is a protecting group for a hydroxy group, following the 2' substitution reaction step, P¹ can be deprotected to convert P¹ into a hydroxy group.

The protection of the 5' position, the 2' substitution reaction and the deprotection of the 5' position of an anhydrouridine compound of formula (I) (compound (I-1)) are represented by the following scheme.

### (Scheme III)

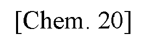

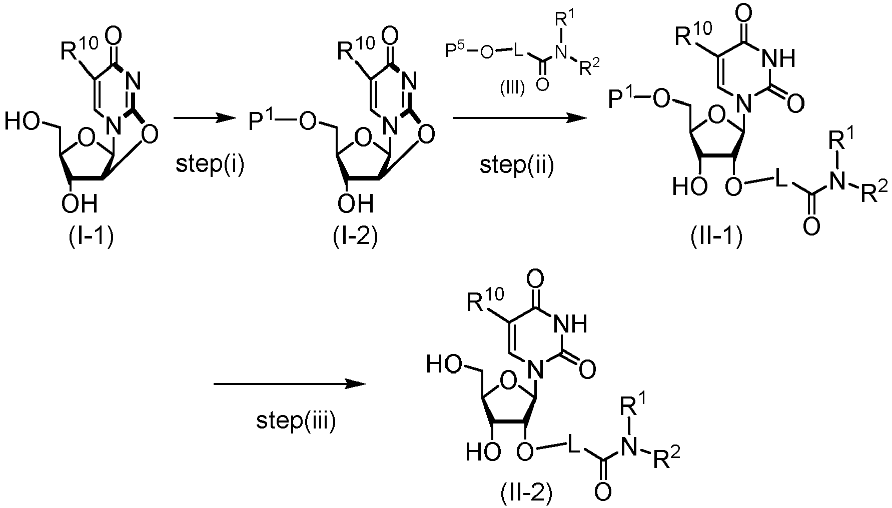

In the scheme, R¹, R², R¹⁰, L, P¹ and P⁵ are the same as described above, and the step (ii) is as described above.

### Step (i)

A compound (I-2) that is the formula (I) compound in which P¹ is a protecting group for a hydroxy group can be obtained by protecting the 5'-hydroxy group in the compound (I-1) that is the formula (I) compound in which P¹ and P² are hydroxy groups.

For the protection of the 5'-hydroxy group, a protecting group that is often used in organic synthetic chemistry (for example, nucleic acid chemistry) can be used, and the hydroxy group can be protected by, for example, a method described in Protective Groups in Organic Synthesis, Fourth edition, written by T. W. Greene, John Wiley & Sons Inc. (2006).

For example, when the protecting group is a silyl-based protecting group (for example, trimethylsilyl group, triisopropylsilyl group, t-butyldimethylsilyl group, t-butyldiphenylsilyl group or the like), the silyl-based protecting group can be introduced into the 5' hydroxy group by reacting a corresponding silylating agent (for example, t-butyldiphenylsilyl chloride) in a solvent under a basic condition (for example, triethylamine, N,N-diisopropylethylamine or pyridine). The solvent is required not to affect the reaction. An amide-based solvent such as N,N-dimethylformamide is preferable. The base itself can also be used as the solvent.

The reaction temperature is preferably 0°C to a solvent reflux temperature and more preferably 20 to 30°C.

The reaction time is, for example, 0.5 to 48 hours, preferably 0.5 to 24 hours and more preferably 0.5 to 4 hours.

The crude product after the reaction can be subject to solid-liquid separation to recover formula (I-2) compound, which is a target product.

### Step (iii)

The deprotection of the hydroxy group at the 5' position of the compound (II-1) can be performed under conditions described in, for example, Protective Groups in Organic Synthesis, Fourth edition, written by T. W. Greene, John Wiley & Sons Inc. (2006).

For example, the deprotection of the hydroxy group at the 5' position can be performed by reacting a reaction substrate with a fluorine compound (for example, ammonium fluoride, tetrabutylammonium fluoride, triethylamine trihydrofluoride or the like) in a solvent. The solvent is preferably an ether-based solvent or an alcoholic-based solvent, more preferably an alcoholic-based solvent and still more preferably methanol. The reaction temperature is preferably -20°C to a solvent reflux temperature, more preferably 0 to 60°C and still more preferably 20 to 40°C. The reaction time is, for example, 0.5 to 48 hours, preferably 1 to 36 hours and more preferably 1 to 24 hours.

The crude product after the reaction can be subjected to solid-liquid separation to recover formula (II-2) compound, which is a target product. Before the solid-liquid separation, the crude product can be purified by slurry purification. The slurry purification can be performed by stirring a suspension of the target product for a certain time.

In the scheme III, as described above, the target product of the step (i) can be collected by the solid-liquid separation, the target product of the step (ii) can be collected by the separation operation, and the target product of the step (iii) can be collected by the slurry purification, respectively.

In addition, as described above, the step (ii) in the scheme can be performed in a solvent under a relatively mild temperature condition (for example, 40 to 110°C, preferably 60 to 100°C, more preferably 80 to 100°C and still more preferably 80 to 95°C), and the target product of the step (ii) can be obtained with a favorable yield.

That is, the present scheme does not require complicated purification by column chromatography and can thus be dominantly used as an industrial production method.

### [Method for producing uridine amidite compound]

In addition, the present invention also relates to a method for producing a uridine amidite compound comprising producing the formula (II-2) compound by the step (ii) or the step (i) and the step (ii), and using the formula (II-2) compound as a starting material.

That is, as shown in the following scheme, a uridine amidite compound of the following formula (V) can be obtained by subsequently performing a step (iv) of converting the 5'-hydroxy group into a protecting group for a hydroxy group such as a triarylmethyl-based protecting group and a step (v) of converting the 3'-hydroxy group into an amidite group with an amidite-forming reagent on the compound of formula (II-2) that is the compound of formula (II) having hydroxy groups at the 3' position and the 5' position.

### (Scheme IV)

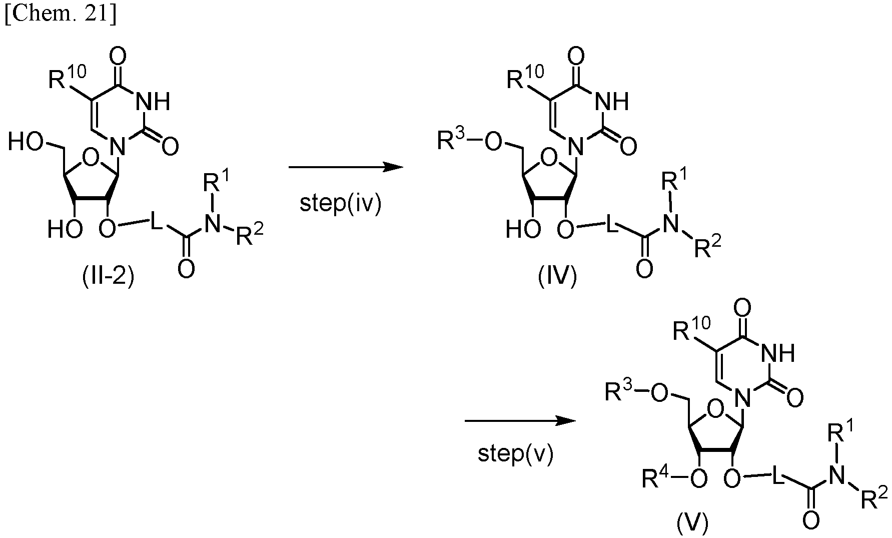

In the scheme, R¹, R², R¹⁰ and L are the same as described above.

R³ is a protecting group for a hydroxy group, preferably a triarylmethyl-based protecting group and particularly preferably 4,4'-dimethoxytrityl group.

R⁴ is the following formula (PA). 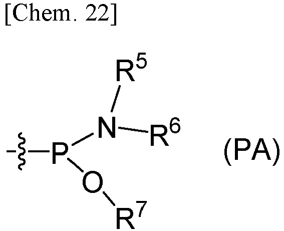
wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group.
R⁵ and R⁶ are particularly preferably i-propyl groups.
R⁷ is particularly preferably a 2-cyanoethyl group.

### Step (iv)

In a step (iv) (the protection of the 5'-hydroxy group), as described above, a protecting group that is often used in organic synthetic chemistry (for example, nucleic acid chemistry) can be used, and the hydroxy group can be protected by, for example, a method described in Protective Groups in Organic Synthesis, Fourth edition, written by T. W. Greene, John Wiley & Sons Inc. (2006).

For example, when R³ is a triarylmethyl-based protecting group (for example, trityl group or 4,4'-dimethoxytrityl group), the triarylmethyl-based protecting group can be introduced into the 5' hydroxy group by reacting a corresponding triarylmethylating agent (for example, trityl chloride or 4,4'-dimethoxytrityl chloride) in a solvent under a basic condition (for example, triethylamine, N,N-diisopropylethylamine or pyridine). The solvent is required not to affect the reaction. A halogenated hydrocarbon-based solvent such as dichloromethane is preferable. The base itself can also be used as the solvent.

The reaction temperature is preferably 0°C to a solvent reflux temperature and more preferably 20 to 30°C.

The reaction time is, for example, 0.5 to 48 hours, preferably 1 to 24 hours and more preferably 1 to 4 hours.

### Step (v)

The present step can be performed under conditions described in, for example, Protective Groups in Organic Synthesis, Fourth edition, written by T. W. Greene, John Wiley & Sons Inc. (2006).

Specifically, the present step can be performed using, for example, an activator (for example, 4,5-dicyanoimidazole, 5-phenyltetrazole or 1H-tetrazole) and an amidite-forming reagent (for example, 2-cyanoethoxy N,N,N',N'-tetraisopropyl phosphordiamidite or 2-cyanoethoxy N,N-diisopropylchlorophosphoramidite). The solvent is required not to affect the reaction. A halogenated hydrocarbon-based solvent such as dichloromethane or a nitrile-based solvent such as acetonitrile is preferable.

The reaction temperature is preferably 0 to 50°C and more preferably 20 to 30°C.

The reaction time is, for example, 0.5 to 48 hours, preferably 1 to 36 hours and more preferably 4 to 24 hours.

### [Method for producing 2'-carbamoylalkyl-modified cytidine compound]

The present invention relates to a method for producing a cytosine compound. Specifically, a cytosine compound of formula (B) is produced by a step (b) of reacting a protected uridine compound of the following formula (A) with a sulfonyl halide compound in a solvent, and then with ammonia water.

### Step (b): Step of converting thymine into cytosine

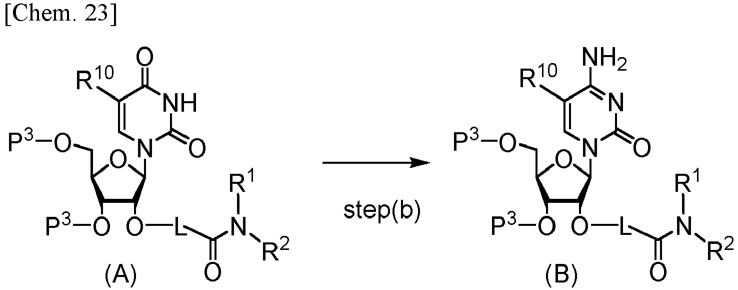

In the scheme, R¹, R², R¹⁰ and L are the same as described above.

P³ are the same as or different from each other and are substitutable benzoyl groups.

P³ are the same as or different from each other and are preferably benzoyl groups that may have one or more substituents selected from the group consisting of a halogen atom, a C₁₋₆ alkyl group and a C₁₋₆ alkoxy group and particularly preferably unsubstituted benzoyl groups.

As the sulfonyl halide compound, for example, a compound of the following formula (C) can be used. 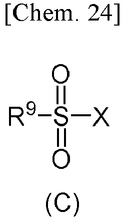 wherein X is a halogen atom, and R⁹ is a substitutable C₁₋₆ alkyl group or a substitutable C₆₋₁₄ aryl group.

When R⁹ is a substitutable C₁₋₆ alkyl group, R⁹ is preferably a C₁₋₆ alkyl group that may have a halogen atom, more preferably a C₁₋₆ alkyl group that may have one to five halogen atoms and still more preferably a C₁₋₆ alkyl group that may have one to five fluorine atoms.
When R⁹ is a substitutable C₆₋₁₄ aryl group, R⁹ is preferably a C₆₋₁₄ aryl group that may have one or more substituents selected from the group consisting of halogen atoms and C₁₋₆ alkyl groups, more preferably a phenyl group that may have one to five substituents selected from the group consisting of halogen atoms and C₁₋₆ alkyl groups and still more preferably a phenyl group that may have one to three C₁₋₆ alkyl groups.

Examples of the sulfonyl halide compound include methanesulfonyl chloride, trifluoromethanesulfonyl chloride, benzenesulfonyl chloride, p-toluenesulfonyl chloride, 2,4,6-trimethylsulfonyl chloride, 2,4,6-triisopropylbenzenesulfonyl chloride, naphthylsulfonyl chloride and the like. The sulfonyl compound that is used in the present step is preferably methanesulfonyl chloride, p-toluenesulfonyl chloride or 2,4,6-triisopropylbenzenesulfonyl chloride and particularly preferably p-toluenesulfonyl chloride.

The amount of the sulfonyl halide compound used is preferably 1.0 to 5.0 molar equivalents, more preferably 2.0 to 4.0 molar equivalents and still more preferably 2.5 to 3.5 molar equivalents, with respect to 1 molar equivalent of the compound (A).

When the protected uridine compound of formula (A) and the sulfonyl halide compound are reacted in the solvent, a base is preferably used. The base is preferably N,N-dimethylaminopyridine, trialkylamine (triethylamine, N,N-diisopropylethylamine or the like) or a combination thereof, more preferably N,N-dimethylaminopyridine and trialkylamine or a combination thereof and particularly preferably a combination of N,N-dimethylaminopyridine and triethyllamine.

The amount of the base used is preferably 1.0 to 20 molar equivalents, more preferably 1.0 to 15 molar equivalents and still more preferably 2.0 to 10 molar equivalents, with respect to 1 molar equivalent of the compound (A). In a case where the combination of N,N-dimethylaminopyridine and trialkylamine is used as the base, the amount of the N,N-dimethylaminopyridine used is preferably 1.0 to 10 molar equivalents, more preferably 1.0 to 5.0 molar equivalents and still more preferably 2.0 to 4.0 molar equivalents with respect to 1 molar equivalent of the compound (A), and the amount of the trialkylamine used is preferably 1.0 to 20 molar equivalents, more preferably 2.0 to 15 molar equivalents and still more preferably 5.0 to 15 molar equivalents with respect to 1 molar equivalent of the compound (A).

The solvent may be a solvent in which the reaction favorably progresses, and examples thereof include nitrile-based solvents, halogen-based solvents (halogenated aromatic hydrocarbon solvents and halogenated hydrocarbon solvents), ether-based solvents, amide-based solvents and the like. A nitrile-based solvent is preferably used.

The reaction temperature between the protected uridine compound of formula (A) and a sulfonyl halide compound is preferably 0 to 100°C, more preferably 10 to 60°C and still more preferably 10 to 30°C.

The reaction time between the protected uridine compound of formula (A) and the sulfonyl halide compound is, for example, 0.5 to 48 hours, preferably 1 to 24 hours and more preferably 2 to 6 hours.

After the reaction with the sulfonyl halide compound, ammonia water is added to the system, and is reacted.

The concentration of the ammonia water to be added may be a concentration at which the reaction favorably progresses and is, for example, preferably 5 to 90 mass%, more preferably 5 to 40 mass%, still more preferably 10 to 30 mass% and particularly preferably 20 to 30 mass%.

The amount of the ammonia water used is preferably 1.0 to 20 molar equivalents, more preferably 5.0 to 15 molar equivalents and still more preferably 8.0 to 9.0 molar equivalents, with respect to 1 molar equivalent of the compound (A).

The reaction temperature with the ammonia water is preferably 0 to 100°C, more preferably 10 to 60°C and still more preferably 10 to 30°C.

The reaction time with the ammonia water is, for example, 0.5 to 48 hours, preferably 1 to 24 hours and more preferably 2 to 6 hours.

Step (a) (step (a1) and (a2)): Step of protecting 3' and 5' (protection of hydroxy group before cytosination)

In the present invention, it is possible to include, before the step (b), a step (a1) of reacting a uridine compound of the following formula (II-2) with a benzoylation reagent to synthesize the protected uridine compound of formula (A) (benzoyl-type protected uridine compound), and then after the step (a1), a step (a2) of obtaining the protected uridine compound of formula (A) by solid-liquid separation.

The present step is characterized in that the synthesized protected uridine compound of formula (A) can be obtained by solid-liquid separation with no need of a complicated purification step such as column chromatography. 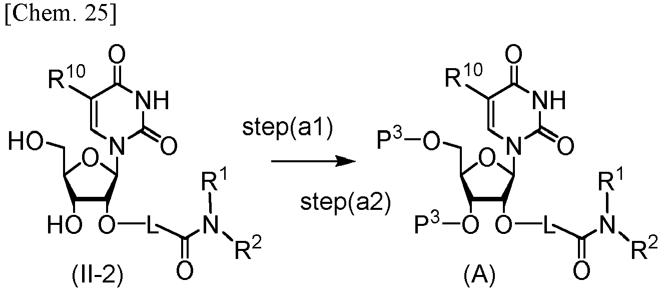 In the scheme, R¹, R², R¹⁰, L and P³ are the same as described above.

The protection with the substituted or unsubstituted benzoyl groups at the 3' position and the 5' position of the compound (II-2) (step (a1)) can be performed under conditions described in, for example, Protective Groups in Organic Synthesis, Fourth edition, written by T. W. Greene, John Wiley & Sons Inc. (2006).

Specifically, the protection step with the benzoyl groups can be performed by, for example, reacting the uridine compound of formula (II-2), which is a reaction substrate, with a substitutable benzoyl halide (for example, benzoyl chloride, p-toluenebenzoyl chloride, p-methoxybenzoyl chloride, 2,4-dimethoxybenzoyl chloride or the like) in a solvent.

The solvent that is used in the present step is preferably a pyridine-based solvent and more preferably pyridine. The reaction temperature is preferably -20°C to a solvent reflux temperature, more preferably 0 to 60°C and still more preferably 20 to 40°C. The reaction time is, for example, 0.5 to 48 hours, preferably 1 to 36 hours and more preferably 1 to 24 hours.

Since a benzoyl-type protected uridine compound in which the 3' position and the 5' position are protected with substitutable benzoyl groups and the 2' position is a substitutable carbamoylalkyl-modified benzoyl-type protected uridine compound (compound (A)) is solid, the benzoyl-type protected uridine compound can be obtained by solid-liquid separation (step (a2)). Therefore, the use of the benzoyl-type protecting groups as the protecting groups for the 3' position and the 5' position makes it possible to reduce complicated purification by column chromatography.

### [Method for producing a cytosine amidite compound and an intermediate thereof]

In addition, the present invention also relates to a method for producing a cytosine amidite compound of formula (G) and an intermediate thereof comprising using, as a starting material, the cytosine compound of formula (B) obtained by the step (b) or the step (a) (the steps (a1) and (a2)) and the step (b).

The entire scheme of the method for producing a cytosine amidite compound is as shown below. 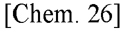 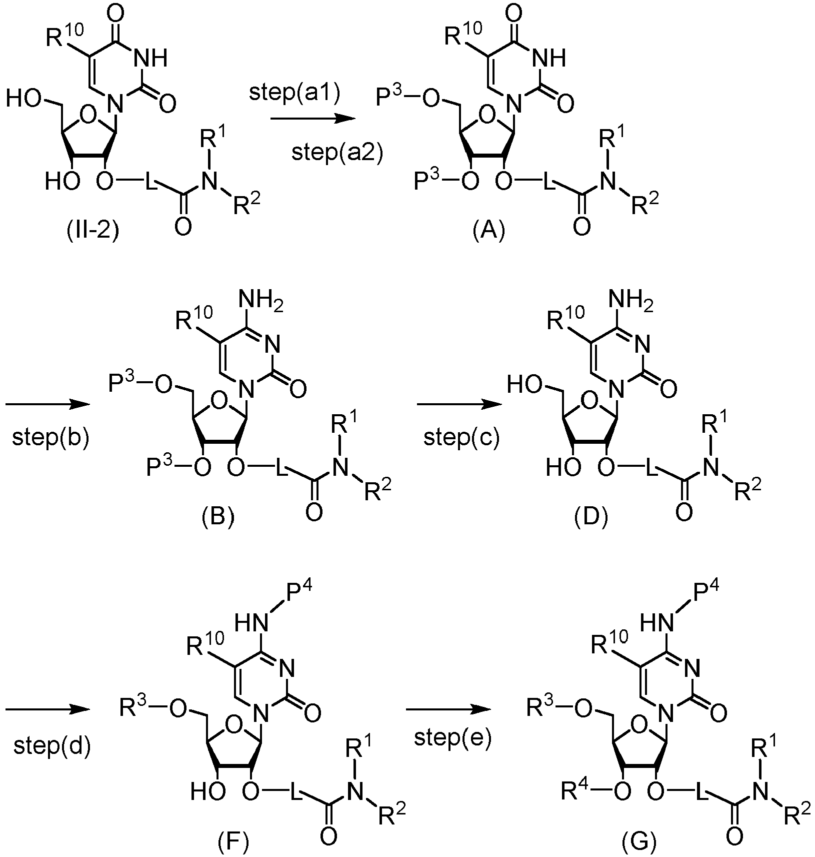 In the scheme, R¹, R², R³, R⁴, R¹⁰, L and P³ are the same as described above, and the steps (a1) and (a2) and the step (b) are as described above.

P⁴ is a protecting group for an amino group, preferably an acyl-based protecting group, more preferably an acetyl group, a propionyl group, an i-butyryl group, a pivaloyl group, a n-butyryl group, a benzoyl group or a phenoxyacetyl group, still more preferably an acetyl group or a benzoyl group and particularly preferably a benzoyl group.

### Step (c): The step of deprotecting 3' position and 5' position

The step (c) is a step of obtaining a cytosine compound of formula (D) by deprotecting benzoyl-type protecting groups in the compound (B) to convert the 3' position and the 5' position into hydroxy groups. 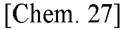 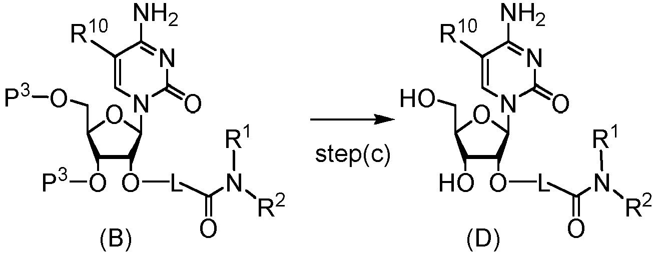 In the scheme, R¹, R², R¹⁰, L and P³ are the same as described above.

The deprotection of the benzoyl-type protecting groups at the 3' position and the 5' position of the compound (B) can be performed under conditions described in, for example, Protective Groups in Organic Synthesis, Fourth edition, written by T. W. Greene, John Wiley & Sons Inc. (2006).

Specifically, the deprotection can be performed by, for example, reacting the compound of formula (B), which is a reaction substrate, with a primary or secondary alkylamine (for example, methylamine, ethylamine, dimethylamine or the like) in a solvent. The primary alkylamine refers to a compound in which one hydrogen atom of ammonia is substituted with an alkyl group (for example, a C₁₋₆ alkyl group), and the secondary alkylamine refers to a compound in which two hydrogen atoms of ammonia are each substituted with an alkyl group (for example, a C₁₋₆ alkyl group). The primary or secondary alkylamine is preferably a primary alkylamine and particularly preferably methylamine.

The solvent is preferably an alcoholic-based solvent and more preferably methanol. The reaction temperature is preferably -20°C to a solvent reflux temperature, more preferably 0 to 60°C and still more preferably 20 to 40°C. The reaction time is, for example, 0.5 to 48 hours, preferably 1 to 36 hours and more preferably 1 to 24 hours.

### Step (d): Step of protecting cytosine base

A step (d) is a step of protecting a 5'-hydroxy group in a compound (D) (step (d1)) and protecting an amino group of the cytosine base (step (d2)). A protected cytosine compound of formula (F) is obtained by performing the present step. 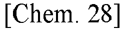 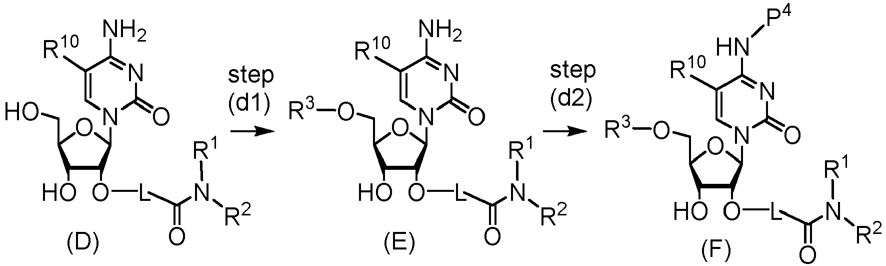 In the scheme, R¹, R², R³, R¹⁰, L and P⁴ are the same as described above.

For the protection of the cytosine base in the compound (D) (step (d2)), as described above, a protecting group that is often used in organic synthetic chemistry (for example, nucleic acid chemistry) can be used, and the cytosine base can be protected by, for example, a method described in Protective Groups in Organic Synthesis, Fourth edition, written by T. W. Greene, John Wiley & Sons Inc. (2006). As the protecting group for the amino group, phenoxyacetyl group, 4-isopropylphenoxyacetyl group, acetyl group, propionyl group, isobutyryl group, pivaloyl group, n-butyryl group, benzoyl group or N,N-dimethylaminomethylene group is preferable, acetyl group or benzoyl group is more preferable, and benzoyl group is particularly preferable.

For example, when the protecting group for the amino group is an acyl-based protecting group (for example, isobutyryl, acetyl, benzoyl or phenoxyacetyl), the amino group can be protected by reacting a corresponding acyl halide (for example, isobutyryl chloride, acetyl chloride, benzoyl chloride or phenoxyacetyl chloride) in a solvent under a basic condition (for example, triethylamine, N,N-diisopropylethylamine or pyridine). The solvent is not particularly limited as long as a protection reaction progresses favorably, an ether-based solvent such as tetrahydrofuran, a halogenated hydrocarbon-based solvent such as dichloromethane, a lower aliphatic acid-based ester solvent such as ethyl acetate or an amide-based solvent such as N,N-dimethylformamide is preferable, an amide-based solvent is more preferable, and N,N-dimethylformamide is more preferable.

The reaction temperature relating to the protection of the amino group is preferably -20 to 20°C and more preferably -10 to 10°C.

The reaction time relating to the protection of the amino group is, for example, 0.5 to 48 hours, preferably 1 to 24 hours and more preferably 1 to 6 hours.

In the present step, the protection of the 5'-hydroxy group in the compound (D) (step (d1)) is collectively performed before the protection of the cytosine base. For the protection of the 5'-hydroxy group, as described above, a protecting group that is often used in organic synthetic chemistry (for example, nucleic acid chemistry) can be used, and the 5' hydroxy group can be protected by, for example, a method described in Protective Groups in Organic Synthesis, Fourth edition, written by T. W. Greene, John Wiley & Sons Inc. (2006).

For example, when the protecting group is a triarylmethyl-based protecting group (for example, trityl group or 4,4'-dimethoxytrityl group), the triarylmethyl-based protecting group can be introduced into the 5' hydroxy group by reacting a corresponding triarylmethylating agent (for example, trityl chloride or 4,4'-dimethoxytrityl chloride) in a solvent under a basic condition (for example, triethylamine, N,N-diisopropylethylamine or pyridine). The solvent is required not to affect the reaction. A halogenated hydrocarbon-based solvent such as dichloromethane is preferable. The base itself can also be used as the solvent.

The reaction temperature is preferably 0°C to a solvent reflux temperature and more preferably 20 to 30°C.

The reaction time is, for example, 0.5 to 48 hours, preferably 1 to 24 hours and more preferably 1 to 4 hours.

### Step (e): Amidite formation step

A step (e) is a step of converting the compound (F) into a cytosine amidite compound of formula (G) with an amidite-forming reagent, that is, a step of converting a 3'-hydroxy group in the compound (F) into phosphoramidite. 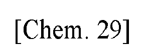 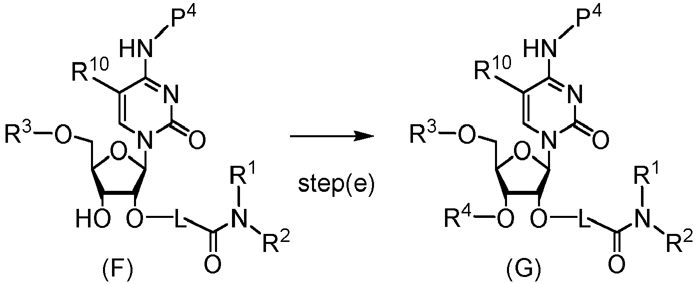 In the scheme, R¹, R², R³, R⁴, R¹⁰, L and P⁴ are the same as described above.

The present step can be performed under conditions described in, for example, Protective Groups in Organic Synthesis, Fourth edition, written by T. W. Greene, John Wiley & Sons Inc. (2006). Specifically, the present step can be performed using, for example, an activator (for example, 4,5-dicyanoimidazole, 5-phenyltetrazole or 1H-tetrazole) and an amidite-forming reagent (for example, 2-cyanoethoxy N,N,N',N'-tetraisopropyl phosphordiamidite or 2-cyanoethoxy N,N-diisopropylchlorophosphoramidite). The solvent is required not to affect the reaction. A halogenated hydrocarbon-based solvent such as dichloromethane or a nitrile-based solvent such as acetonitrile is preferable.

The reaction temperature is preferably 0 to 50°C and more preferably 20 to 30°C.

The reaction time is, for example, 0.5 to 48 hours, preferably 1 to 36 hours and more preferably 4 to 24 hours.

Examples of the cytosine amidite compound of formula (G) include N4-(benzoyl)-5'-O-(4,4'-dimethoxytrityl)-2'-O-[2-(N-methylcarbamoyl)ethyl]-5-methylcytidine-3'-(2-cyanoethyl N,N-diisopropylphosphoramidite) and N4-(acetyl)-5'-O-(4,4'-dimethoxytrityl)-2'-O-[2-(N-methylcarbamoyl)ethyl]-5-methylcytidine-3'-(2-cyanoethyl N,N-diisopropylphosphoramidite).

### Examples

Hereinafter, examples will be described, and the present invention will be described in more detail, but the present invention is not limited to these examples.

In reference synthesis examples and the examples, "HPLC" means high-performance liquid chromatography, "LC-MS" means liquid chromatography-mass spectrometry, and "NMR" means nuclear magnetic resonance. "(v/v)" means (volume/volume). In each chromatography and experiment operation, "number/number" means the volume ratio of each solvent unless particularly otherwise described.

In the examples, unless particularly described, Purif-Pack (SI-50 µm) manufactured by Shoko Science Co., Ltd. was used for purification by silica gel column chromatography.

In a case where ¹H-NMR data are described, the data are measured at 300MHz (JNM-ECP300; manufactured by JEOL Ltd. or JNM-ECX300: manufactured by JEOL Ltd.), and the chemical shifts δ (unit: ppm) of signals for which tetramethylsilane is used as the internal standard (splitting pattern, integral values) are shown. "s" means singlet, "d" means doublet, "t" means triplet, "dd" means doublet of doublets, "m" means multiplet, "brs" means broad singlet, "CDCl₃" means deuterated chloroform and "DMSO-d₆" means deuterated dimethyl sulfoxide, and "D₂O" means deuterated water.

Unless particularly otherwise described, mas (MS or MASS) was measured using an electrospray ionization (ESI) method under the following conditions. "ESI+" means the ESI positive ion mode, and "ESI-" means the ESI negative ion mode.

### <LC-MS analysis conditions>

High-performance liquid chromatography: HPLC manufactured by SHIMADZU CORPORATION
Column: InfinityLab Poroshell 120 EC-C18 (1.9 µm, 2.1 × 50 mm) manufactured by Agilent Technologies, Inc.
Column oven temperature: 40°C
Eluent: A: 10 mM ammonium acetate aqueous solution
B: Acetonitrile
A/B: 90/10 (0 to 1 minute), 90/10 to 5/95 (1 to 3 minutes), 5/95 (3 to 5 minutes) (volume ratio)
Eluent rate: 0.5 mL/minute
Detection wavelength: 210 nm

Unless particularly otherwise described, the HPLC relative purity was measured under the following conditions and was calculated by an area percentage method.

### <HPLC analysis conditions>

High-performance liquid chromatography: HPLC manufactured by SHIMADZU CORPORATION
Column: Triart C18 (5 µm, 4.6 × 250 mm) manufactured by YMC CO., LTD.
Column oven temperature: 40°C
Eluent: A: 10 mM ammonium acetate aqueous solution
B: Acetonitrile
Analysis conditions of intermediate
A/B: 95/5 (0 to 15 minute), 95/5 to 5/95 (15 to 25 minutes), 5/95 (25 to 30 minutes)
(volume ratio)
Analysis conditions of amidite compound
A/B: 50/50 (0 to 5 minute), 50/50 to 5/95 (5 to 15 minutes), 5/95 (15 to 25 minutes), 50/50 (25 to 30 minutes) (volume ratio)
Eluent rate: 1.0 mL/minute
Detection wavelength: 254 nm

Unless particularly otherwise described, powder X-ray diffraction was measured under the following conditions.

### <X-ray diffraction conditions>

X-ray diffractometer: MiniFlex600 manufactured by Rigaku Corporation Radiation source: Cukα
Measurement range: 2θ, 3° to 40°

### [Reference Synthesis Example 1]

### Synthesis of 3-hydroxy-N-methylpropanamide

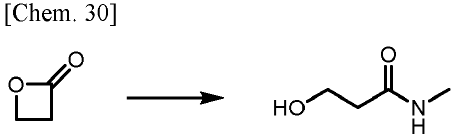

A methylamine methanol solution (191 mL) was cooled to -40°C and stirred, and β-propiolactone (39 mL) was added dropwise thereto over 30 minutes. After 4 hours of stirring, the solution was heated up to room temperature. The reaction solution was vacuum-distilled to obtain a crude product of the title compound (yellow oil, 76.6 g).

### [Reference Synthesis Example 2]

### Synthesis of 3-[(trimethylsilyl)oxy]-N-methylpropanamide

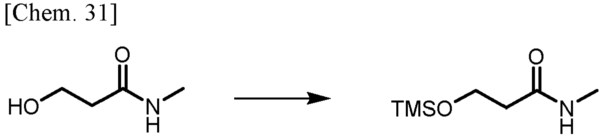

A solution of 3-hydroxy-N-methylpropanamide (52.0 g) in dichlromethane (520 mL) was cooled with ice, and triethylamine (80.4 mL) was added thereto. Chlorotrimethylsilane (66.9 mL) was added dropwise to the reaction solution over 30 minutes. The solution was heated up to room temperature, and after 1 hour of stirring, diisopropyl ether (520 mL) was added thereto, and after the solution was cooled with ice, the precipitate was filtered. The filtrate was concentrated under reduced pressure to obtain a crude product of the title compound (yellow oil, 94.1 g).

### [Example 1]

### Step 1: Synthesis of 2'-O-[2-(N-methylcarbamoly)ethyl]-5-methyluridine (step (ii))

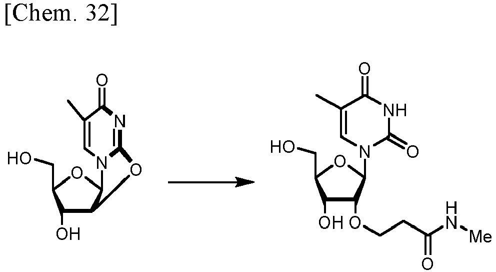

2,2'-O-Anhydro-5-methyluridine (3.0 g, 12.5 mmol) and a boron trifluoride diethyl ether complex (4.71 mL, 37.5 mmol) were added to an ice-cooled solution of 3-[(trimethylsilyl)oxy]-N-methylpropanamide (13.2 g, 75.0 mmol) in N,N-dimethylacetamide (15.0 mL). The solution was stirred at 100°C for seven hours and then returned to room temperature. Water (50 mL) was added to the reaction solution, followed by washing with chloroform (100 mL) twice. Ammonium sulfate (25 g) and water (50 mL) were added to the aqueous phase and extracted with tetrahydrofuran (100 mL). The organic layer was dried over magnesium sulfate and distilled away under reduced pressure, and the obtained residue was loaded onto a silica gel chromatography and eluted with chloroform/methanol (9/1 → 4/1, v/v) to obtain an amorphous substance of the title compound (2.93 g, yield: 68%). 1.0 g of the title compound was dissolved in methanol (5.0 mL), heated to 60°C and slowly cooled to 0°C. The precipitated white solid was filtered and washed with ice-cooled methanol (2.0 mL) twice. The white solid was dried under reduced pressure to obtain the title compound (723 mg, collection rate: 72%). It was confirmed by powder X-ray diffraction that the obtained white solid was a crystal. ¹H-NMR (D₂O) δ: 7.58 (1H, t, J = 1.2 Hz), 5.80 (1H, t, J = 5.1 Hz), 4.21 (1H, t, J = 5.7 Hz), 4.02 (1H, t, J=5.1 Hz), 3.99-3.96 (1H, m), 3.86-3.65 (4H, m), 2.58 (3H, s), 2.40 (2H, m), 1.78 (3H, d, J = 1.2 Hz)
MASS (ESI+) m/z; 343.95 (M + H) +
Characteristic peaks of powder X-ray diffraction; 6.6, 10.4, 15.2, 19.0, 19.5 and 23.4

### Step 2: Synthesis of 5'-O-(4,4'-dimethoxytrityl)-2'-O-[2-(N-methylcarbamoly)ethyl]-5-methyluridine (step (iv))

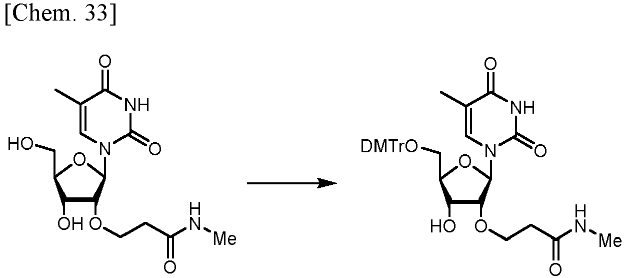

N,N-diisopropylethylamine (495 µL, 2.92 mmol) and 4,4'-dimethoxytrityl chloride (543 mg, 1.61 mmol) were added to a solution of 2'-O-[2-(N-methylcarbamoly)ethyl]-5-methyluridine (500 mg, 1.46 mmol) in pyridine (5.0 mL). After 16.5 hours of stirring at room temperature, 4,4'-dimethoxytrityl chloride (98.9 mg, 0.292 mmol) was added thereto, and the solution was further stirred for 3 hours. Methanol (2 mL) was added to a reaction mixture and stirred, and ethyl acetate (10 mL) was then added thereto. The organic layer was sequentially washed with a saturated sodium bicarbonate aqueous solution, water, a saturated sodium bicarbonate aqueous solution and a saturated sodium chloride aqueous solution, and the organic layer was dried over sodium sulfate. The solvent was distilled away under reduced pressure, and the obtained residue was loaded onto a silica gel chromatography, eluted with chloroform/methanol (20/1, v/v) containing 1 vol% of triethylamine and concentrated under reduced pressure, thereby obtaining the title compound (893 mg, yield: 95%).
¹H-NMR (DMSO-d₆) δ: 7.85 (1H, d, J = 4.8 Hz), 7.47-7.21 (10H, m), 6.90 (4H, d, J = 8.5 Hz), 5.81 (1H, d, J = 5.1 Hz), 5.28 (1H, d, J = 5.4 Hz), 4.31-4.26 (1H, m), 4.11-4.05 (1H, m), 3.95 (1H, brs), 3.85-3.74 (8H, m), 3.33-3.16 (3H, m), 2.56 (3H, d, J = 4.8 Hz), 2.39-2.35 (1H, m), 1.40 (3H, d, J = 0.9 Hz)
MASS (ESI-) m/z; 644.30

### Step 3: 5'-O-(4,4'-dimethoxytrityl)-2'-O-[2-(N-methylcarbamoly)ethyl]-5-methyluridine-3'-(2-cyanoethyl N,N-diisopropylphosphoramidite) (step (v))

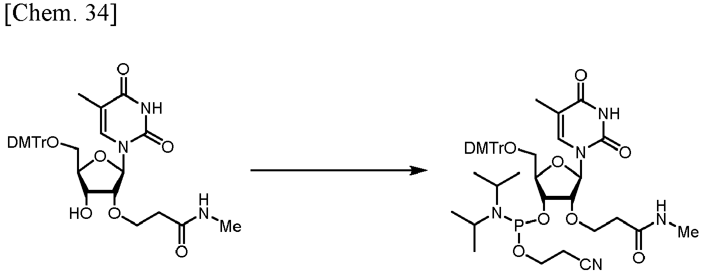

4,5-Dicyanoimidazole (97.8 mg, 0.928 mmol) and 2-cyanoethyl N,N,N',N'-tetraisopropylphosphordiamidite (788 µL, 2.48 µmol) were added to a solution of 5'-O-(4,4'-dimethoxytrityl)-2'-O-[2-(N-methylcarbamoly)ethyl]-5-methyluridine (893 mg, 1.38 mmol) in acetonitrile (1.8 mL)/dichloromethane (5.4 mL). After 16 hours of stirring, ethyl acetate (20 mL) was added to the reaction solution. The organic layer was washed with a saturated sodium bicarbonate aqueous solution (10 mL) and a saturated sodium chloride aqueous solution (10 mL), and the organic layer was dried over sodium sulfate. A residue obtained by distilling the solvent away under reduced pressure was loaded onto a silica gel chromatography, eluted with chloroform/acetone (4/1 → 1/1, v/v) containing 0.5 vol% of triethylamine and concentrated under reduced pressure, thereby obtaining the title compound (870 mg, yield: 75%).
MASS (ESI+) m/z; 846.60

### [Synthesis Example 1]

### Synthesis of 5'-O-(tert-butyldiphenylsilyl)-2,2'-O-[2-(N-methylcarbamoyl)ether]-5-methyluridine (step (i): Protection of 5')

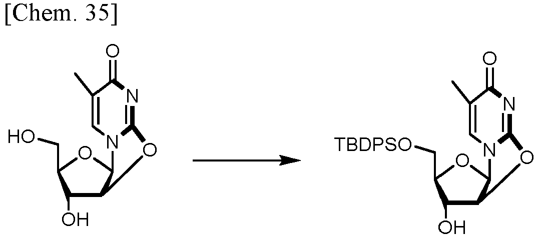

A suspension of 2,2'-O-anhydro-5-methyluridine (42.0 g, 175 mmol) in pyridine (420 mL) and N,N-dimethylformamide (210 mL) were stirred at 60°C to produce a homogeneous solution. The solution was returned to room temperature, and tert-butyldiphenylsilyl chloride (40 g, 146 mmol) was added dropwise thereto over 8 minutes. After 30 minutes of stirring, the reaction solution was added to water (820 mL) over 1 hour. The precipitated solid was filtered, and then washed with water (40 mL) and n-hexane (40 mL), followed by drying under reduced pressure. The obtained white solid (63.4 g) was suspended in ethyl acetate/diisopropyl ether (1/1, 507 mL), stirred for 1 hour and then filtered. The resulting solid was dried to obtain the title compound (53.3 g). ¹H-NMR (DMSO-d₆) δ: 7.78 (1H, d, J = 1.4 Hz), 7.54-7.34 (10H, m), 6.30 (1H, d, J = 5.8 Hz), 5.97 (1H, d, J = 4.4 Hz), 5.21 (1H, dd, J = 5.7, 1.5 Hz), 4.40 (1H, brs), 4.19-4.14 (1H, m), 3.56 (1H, dd, J = 11.7, 4.8 Hz), 3.40 (1H, dd, J= 11.4, 6.9), 1.75 (3H, d, J=1.2 Hz), 0.90 (9H, s)
MASS (ESI+) m/z; 479.15

### [Example 2]

### Synthesis of 5'-O-(tert-butyldiphenylsilyl)-2'-O-[2-(N-methylcarbamoly)ethyl]-5-methyluridine (step (ii))

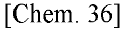

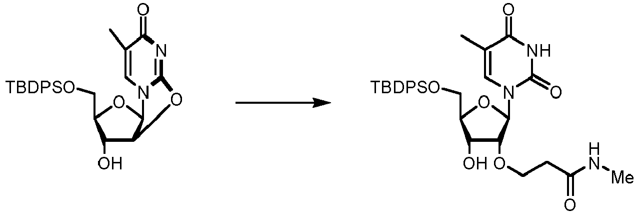

A boron trifluoride diethyl ether complex (356 mg, 2.51 mmol) was added to a solution of 3-[(trimethylsilyl)oxy]-*N*-methylpropanamide (0.879 g, 5.01 mmol) in dioxane (6.0 mL) and stirred at room temperature for 30 minutes, and then 5'-O-(tert-butyldiphenylsilyl)-2,2'-O-[2-(N-methylcarbamoyl)ether]-5-methyluridine (600 mg, 1.25 mmol) was added thereto. The solution was stirred for 6 hours at 90°C and then returned to room temperature. Ethyl acetate (12 mL) was added to the reaction solution, and the solution was washed with water (6 mL) and a 10% sodium chloride aqueous solution (6 mL) and then concentrated under reduced pressure to obtain a crude product of the title compound (729 mg).
MASS (ESI+) m/z; 582.30 (M + H) +

### [Example 3]

### Synthesis of 5'-O-(tert-butyldiphenylsilyl)-2'-O-[2-(N-methylcarbamoly)ethyl]-5-methyluridine (step (ii))

The reaction conditions of Example 2 were verified. Specifically, with respect to 50 mg of a uridine compound that was a substrate, solvents shown in Table 1 were verified using 4.0 equivalents of a silane compound and 2.0 equivalents of a boron trifluoride diethyl ether complex. As the amounts of the solvents, 0.5 mL was employed. The results obtained by calculating the conversion rate after 16 hours from the addition of the uridine compound which was a raw material are shown in the following table. In the formula, "BF₃·OEt₂" means a boron trifluoride diethyl ether complex, and "Solvent" means a solvent. In Table 1, "solvent used (10 vol.)" means that 0.5 mL of a solvent was used with respect to 0.050 g of a substrate (0.5 (mL)/0.05 (g) = 10).

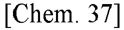

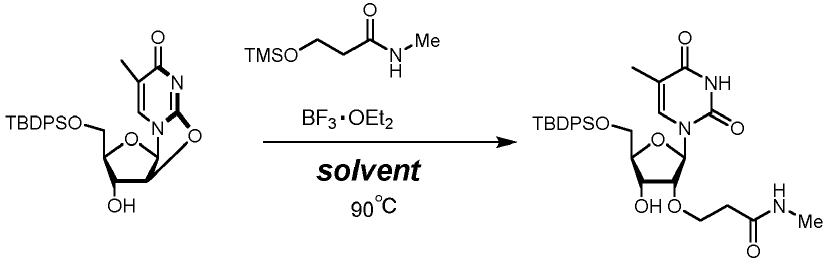

### [Table 1]

**[Table 1]**

| Solvent used (10 vol.) | Conversion rate into target product (16 h) |
|---|---|
| Ethyl acetate | Trace amount |
| Acetonitrile | 4% |
| Tetrahydrofuran | 88% |
| Chloroform | 51% |
| 4-Methyltetrahydropyran | 99.5% |
| Dioxane | 99.5% |
| 1,2-Dimethoxyethane | 98.8% |
| Pyridine | 11.3% |

As shown in Table 1, the results are as described below: in cases where dioxane, 4-methyltetrahydrofuran, 1,2-dimethoxyethane were used as solvents, the conversion rates into the target product exceeded 98%, and, in a case where tetrohydrofuran was used as a solvent, the conversion rate exceeded 85%. Among them, with dioxane, an oil out phenomenon was not exhibited during the reaction, and the time taken for the reaction to be completed was 6 hours.

### [Synthesis Example 2]

### Synthesis of 2'-O-[2-(N-methylcarbamoyl)ether]-5-methyluridine (step (iii): deprotection of 5')

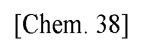

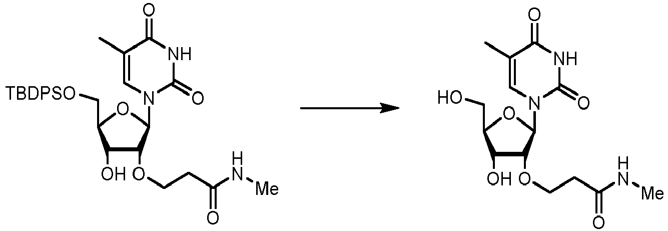

Ammonium fluoride (13.9 g, 138 mmol) was added to a solution of 5'-O-(tert-butyldiphenylsilyl)-2'-O-[2-(N-methylcarbamoly)ethyl]-5-methyluridine (40.0 g) in methanol (600 mL). After 4 hours of stirring at 60°C, the reaction solution was concentrated, water (600 mL) and ethyl acetate (400 mL) were added thereto for washing, and then tetrahydrofuran (600 mL) and ammonium sulfate (310 g) were added to the aqueous phase to extract the title compound into the organic layer. The organic layer was dried over sodium sulfate and concentrated under reduced pressure to obtain a solid (25.1 g). The solid (25.1 g) was suspended in methanol (250 mL), stirred at 50°C for 2 hours, and thereafter cooled to zero degrees over 5 hours and then filtered. The resulting solid was dried to obtain the title compound (16.0 g, yield: 68%). It was confirmed by powder X-ray diffraction that the white solid was a crystal.
¹H-NMR (D₂O) δ: 7.58 (1H, t, J = 1.2 Hz), 5.80 (1H, t, J = 5.1 Hz), 4.21 (1H, t, J = 5.7 Hz), 4.02 (1H, t, J = 5.1 Hz), 3.99-3.96 (1H, m), 3.86-3.65 (4H, m), 2.58 (3H, s), 2.40 (2H, m), 1.78 (3H, d, J=1.2 Hz)
MASS (ESI+) m/z; 343.95
Characteristic peaks of powder X-ray diffraction; 6.6, 10.4, 15.2, 19.0, 19.5 and 23.4

### [Synthesis of MCE 5mC (Bz) amidite]

### [Example 4]

### Synthesis of 3',5'-di-O-(benzoyl)-2'-O-[2-(N-methylcarbamoly)ethyl]-5-methyluridine (steps (a1) and (a2))

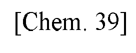

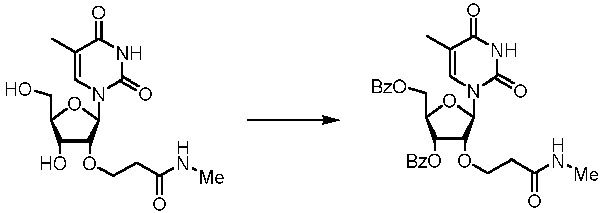

Benzoyl chloride (1.23 mL, 10.70 mmol) was added dropwise to an ice-cooled solution of 2'-O-[2-(N-methylcarbamoly)ethyl]-5-methyluridine (1.67 g, 4.86 mmol) in pyridine (16.7 mL) and stirred for 30 minutes. Ethyl acetate (20 mL) was added to the reaction solution, and then the solution was washed with a saturated sodium bicarbonate aqueous solution. The organic layer was dried over magnesium sulfate, and then toluene was added thereto and distilled away under reduced pressure to obtain a white solid (2.70 g). The white solid was suspended in ethyl acetate (27 mL), heated to 60°C, and then stirred at room temperature for 30 minutes and filtered. The resultant was dried to obtain a crystal (1.93 g, yield: 72%) of the title compound. The solid (772 mg) obtained by concentrating a filtrate was suspended in ethyl acetate (5.0 mL), stirred for 30 minutes and filtered. The resulting solid was dried to obtain a total of 2.27 g (yield: 85%) of the crystal of the title compound. It was confirmed by powder X-ray diffraction that the white solid was a crystal.
¹H-NMR (CDCl₃) δ: 8.28 (1H, s), 8.09-8.04 (4H, m), 7.77-7.59 (2H, m), 7.51-7.46 (4H, m), 7.23 (1H, d, J = 1.4 Hz), 6.05 (1H, d, J= 4.4 Hz), 5.89 (1H, brs), 5.48 (1H, t, J = 5.7 Hz), 4.83 (1H, dd, J = 12.3, 2.7 Hz), 4.68-4.64 (1H, m), 4.59 (1H, dd, J = 12.3, 3.3 Hz), 4.33 (1H, t, J = 4.9 Hz), 3.90-3.86 (2H, m), 2.59 (3H, d, J= 4.8 Hz), 2.39-2.33 (2H, m), 1.62 (3H, d, J = 0.9 Hz)
MASS (ESI+) m/z; 552.25
Characteristic peaks of powder X-ray diffraction; 6.3, 11.1, 12.3, 16.5, 17.4 and 20.6

### [Example 5]

### Synthesis of 5'-O-(4,4'-dimethoxytrityl)-2'-O-[2-(N-methylcarbamoyl)ethyl]-5-methylcytidine (step (b), step (c) and step (d1))

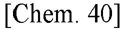

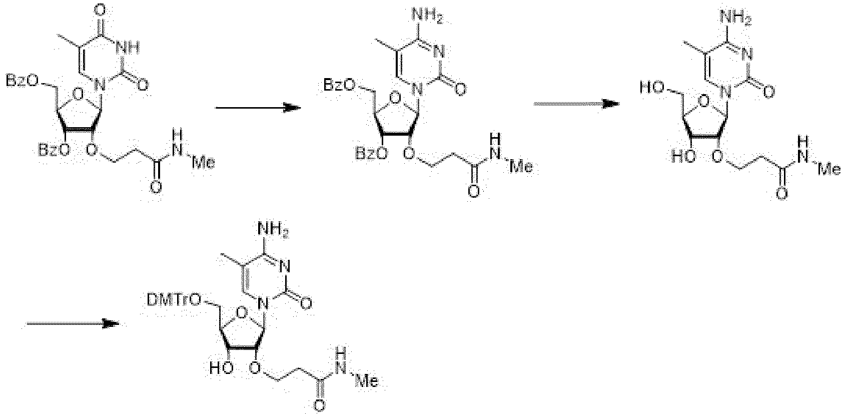

### 1) Step (b)

Triethylamine (9.55 mL, 68.5 mmol) and N-dimethylaminopyridine (2.51 g, 20.6 mmol) were added to a solution of 3',5'-di-O-(benzoyl)-2'-O-[2-(N-methylcarbamoly)ethyl]-5-methyluridine (3.79 g, 6.85 mmol) in acetonitrile (76 mL) and then cooled with ice, and N,N-dimethylaminopyridine (418 mg, 3.43 mmol) and p-toluenesulfonyl chloride (3.92 g, 20.6 mmol) were added thereto. After 2 hours of stirring at room temperature, 28% ammonia water (40 mL) was added thereto, and the solution was stirred for 1.5 hours. Ethyl acetate (150 mL) was added to the reaction solution, the solution was washed with 1 M hydrochloric acid (50 mL) twice and then washed with a saturated sodium bicarbonate aqueous solution (50 mL), and the organic layer was dried over magnesium sulfate. The organic layer was concentrated under reduced pressure, and an oil (8.3 g) containing 3',5'-di-O-(benzoyl)-2'-O-[2-(N-methylcarbamoyl)ethyl]-5-methylcytidine was obtained.

### 2) Step (c)

A 9.8 M methylamine-methanol solution (3.0 mL) was added to a solution of the oil (988 mg) obtained in 1)} in methanol (5 mL). The solution was stirred at room temperature for 1 hour and then concentrated under reduced pressure. Water (5 mL) was added thereto, followed by washing with ethyl acetate (10 mL). Acetonitrile was added thereto, and the solution was concentrated under reduced pressure to obtain a crude product (388 mg) of 2'-O-[2-(N-methylcarbamoyl)ethyl]-5-methylcytidine.

### 3) Step (d1)

N,N-diisopropylethylamine (2.33 mL) and 4,4'-dimethoxytrityl chloride (2.55 g) were added to a solution of 2'-O-[2-(N-methylcarbamoyl)ethyl]-5-methylcytidine (2.23 g) in pyridine (22 mL). After 1.5 hours of stirring at room temperature, methanol (2.0 mL) was added thereto, and the solution was further stirred for 1 hour. Ethyl acetate (100 mL) was added to the reaction solution, and the solution was washed with a saturated sodium bicarbonate aqueous solution (50 mL) and a saturated sodium chloride aqueous solution (20 mL). The organic layer was dried over sodium sulfate, and then the solvent was distilled away under reduced pressure, and the obtained residue was loaded onto a silica gel chromatography (CHROMATOREX NH-DM1020) and eluted with chloroform/methanol (30/1, v/v) containing 0.5 vol% of triethylamine to obtain an oil (3.41 g, yield in the three steps: 77%) of the title compound.
¹H-NMR (DMSO-d₆) δ: 7.89-7.84 (1H, m), 7.45-7.23 (10H, m), 6.89 (4H, d, J = 9.0 Hz), 5.81 (1H, d, J = 3.6 Hz), 5.21 (1H, brs), 4.24 (1H, brs), 3.93-3.86 (2H, m), 3.81-3.77 (2H, m), 3.72 (6H, s), 2.55 (3H, d, J = 4.5 Hz), 2.42-2.28 (2H, m), 1.41 (3H, s)
MASS (ESI-) m/z; 643.30

### [Example 6]

### Synthesis of N4-(benzoyl)-5'-O-(4,4'-dimethoxytrityl)-2'-O-[2-(N-methylcarbamoyl)ethyl]-5-methylcytidine (step (d2))

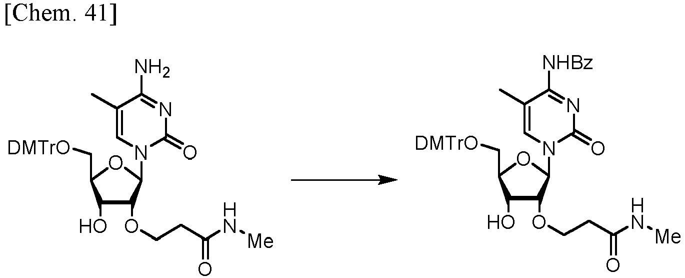

Benzoic anhydride (1.19 g, 5.28 mmol) was added to a solution of 5'-O-(4,4'-dimethoxytrityl)-2'-O-[2-(N-methylcarbamoyl)ethyl]-5-methylcytidine (3.41 g, 5.28 mmol) in N,N-dimethylformamide (68 mL). After 16 hours of stirring at room temperature, ethyl acetate (340 mL) was added to the reaction solution, and the solution was washed with a saturated sodium bicarbonate aqueous solution (100 mL). The organic layer was dried over magnesium sulfate, and then the solvent was distilled away under reduced pressure, and the obtained residue was loaded onto a silica gel chromatography and eluted with chloroform/methanol (1/0 → 4/1, v/v) containing 1 vol% of triethylamine to obtain an oil of the title compound (2.11 g, yield: 53%). Acetonitrile (18 mL) was added to the obtained oil (2.11 g), and the oil was solidified. A suspension was filtered and then dried under reduced pressure to obtain a white solid (1.67 g, collection rate: 79%). It was confirmed by powder X-ray diffraction that the white solid was a crystal.
¹H-NMR (DMSO-d₆) δ: 8.11 (1H, brs), 7.83-7.76 (2H, m), 7.59-7.20 (12H, m), 6.96-6.82 (4H, m), 5.81 (1H, d, J = 3.9 Hz), 5.32 (1H, d, J = 6.3 Hz), 4.30 (1H, m), 4.08 (1H, brs), 3.97 (1H, brs), 3.80 (2H, m), 3.68 (6H, s), 2.56 (3H, d, J= 4.5 Hz), 2.42-2.31 (2H, m), 1.60 (3H, s)
MASS (ESI+) m/z; 749.40
Characteristic peaks of powder X-ray diffraction; 5.6, 10.8, 13.2, 14.4, 17.2, 18.9 and 21.1

### [Example 7]

### Synthesis of N4-(benzoyl)-5'-O-(4,4'-dimethoxytrityl)-2'-O-[2-(N-methylcarbamoyl)ethyl]-5-methylcytidine-3'-(2-cyanoethyl N,N-diisopropylphosphoramidite) (step (e))

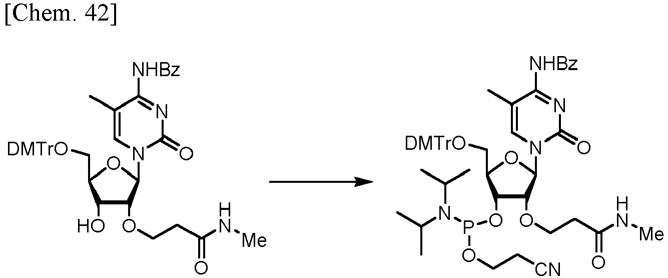

2-Cyanoethyl N,N,N',N'-tetraisopropylphosphordiamidite (63.3 µL, 200 µmοl) was added to a solution of N4-(benzoyl)-5'-O-(4,4'-dimethoxytrityl)-2'-O-[2-(N-methylcarbamoyl)ethyl]-5-methylcytidine (100 mg, 133 µmol) in acetonitrile (1.5 mL)/dichloromethane (500 µL) and cooled with ice. Pyridine-trifluoroacetate (30.8 mg, 160 µL) was added to the reaction solution, and the solution was stirred for 2 hours. Sodium hydrogen carbonate (100 mg) and toluene (3.0 mL) were added thereto, followed by washing with N,N-dimethylformamide/water (1/1, 2.0 mL) four times, washing with water (2.0 mL) twice, and washing with a saturated sodium chloride aqueous solution once. The organic layer was dried over sodium sulfate, and then the solvent was distilled away under reduced pressure, and the obtained residue was loaded onto a silica gel chromatography (CHROMATOREX NH-DM1020) and eluted with n-hexane/ethyl acetate (1/1 → 1/4, v/v) to obtain the title compound (77 mg, yield: 61%).
MASS (ESI+) m/z; 847.50

### [Example 8]

### Synthesis of N4-(acetyl)-5'-O-(4,4'-dimethoxytrityl)-2'-O-[2-(N-methylcarbamoyl)ethyl]-5-methylcytidine (step (d2))

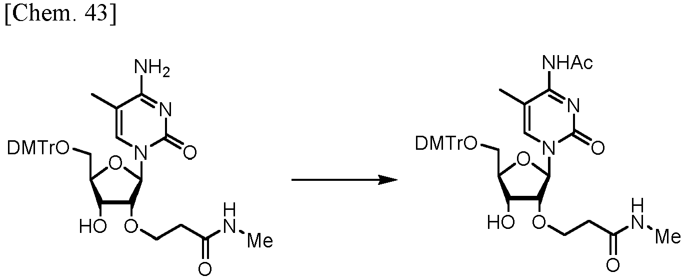

Acetic anhydride (0.25 g, 2.45 mmol) was added to a solution of 5'-O-(4,4'-dimethoxytrityl)-2'-O-[2-(N-methylcarbamoyl)ethyl]-5-methylcytidine (1.05 g, 1.63 mmol) in N,N-dimethylformamide (5.17 g). After 24 hours of stirring at room temperature, ethyl acetate (21.02 g) was added to the reaction solution, and separation was performed by adding a saturated sodium bicarbonate aqueous solution (10.51 g) and water (10.50 g). After the aqueous phase was extracted with ethyl acetate (20.80 g), the organic layer was collectively dried over sodium sulfate, and the solvent was then distilled away under reduced pressure to obtain an oil (1.18 g) of the title compound.
¹H-NMR (DMSO-d₆) δ: 9.82 (1H, brs), 7.87-7.83 (2H, m), 7.40-7.38 (2H, m), 7.33-7.30 (2H, m), 7.27-7.23 (5H, m), 6.91-6.87 (4H, m), 5.79 (1H, d, J= 2.4 Hz), 5.27 (1H, d, J = 6.8 Hz), 4.30-4.25 (1H, m), 4.01-3.98 (1H, m), 3.96-3.94 (1H, m), 3.91-3.80 (2H, m), 3.73 (6H, s), 3.29-3.24 (2H, m), 2.55 (3H, d, J = 4.8 Hz), 2.41-2.34 (2H, m), 2.23 (3H, s), 1.51 (3H, s)
MASS (ESI-) m/z; 685.30

### [Example 9]

### Synthesis of N4-(acetyl)-5'-O-(4,4'-dimethoxytrityl)-2'-O-[2-(N-methylcarbamoyl)ethyl]-5-methylcytidine-3'-(2-cyanoethyl N,N-diisopropylphosphoramidite) (step(e))

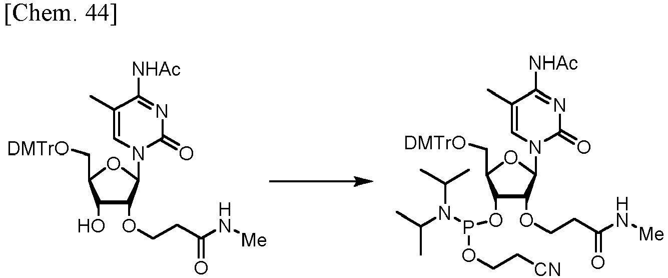

2-Cyanoethyl N,N,N',N'-tetraisopropylphosphordiamidite (0.73 g, 2.42 mmol) was added to a solution of N4-(acetyl)-5'-O-(4,4'-dimethoxytrityl)-2'-O-[2-(N-methylcarbamoyl)ethyl]-5-methylcytidine (crude: 1.18 g) in dichloromethane (11.14 g) and cooled with ice. 4,5-Dicyanoimidazole (145.5 mg, 1.23 mmol) was dissolved in acetonitrile (2.24 g) and added to the reaction solution. After 3 hours of stirring at room temperature, toluene (11.28 g) and acetonitrile (4.50 g) were added thereto, followed by washing with 5% sodium hydrogen carbonate aqueous solutions (11.60 g and 11.05 g) twice. The organic layer was dried over sodium sulfate, and then the solvent was distilled away under reduced pressure, and the obtained residue was loaded onto a silica gel chromatography (Yamazen's Hi-Flash column) and eluted with chloroform/acetone (100/0 → 60/40, v/v) containing 1 vol% of triethylamine. Fractions containing the title compound were combined and concentrated under reduced pressure, and then the obtained residue was loaded onto a silica gel chromatography (Yamazen's Hi-Flash column) and eluted with chloroform/acetone (100/0 → 80/20, v/v) containing 1 vol% of triethylamine to obtain the title compound (543.1 mg, yield: 38%) (two step yield of Examples 8 and 9).
MASS (ESI-) m/z; 885.39

### INDUSTRIAL APPLICABILITY

The present invention makes it possible to provide a novel method for producing a nucleoside.

## Claims

1. A method for producing a uridine compound of the following formula (II): 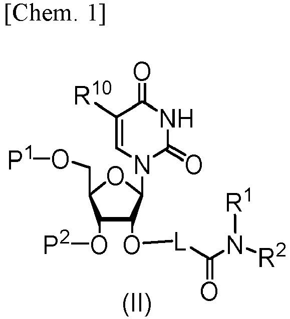 wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, and P¹ and P² are the same as or different from each other and are hydrogen atoms or protecting groups for a hydroxy group,
the method comprising:
a 2' substitution reaction step (ii) of reacting an anhydrouridine compound of the following formula (I) with an amide compound of the following formula (III) in a solvent in the presence of a boron reagent, 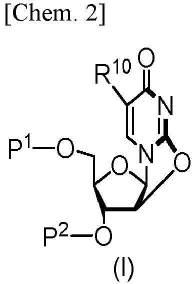
wherein R¹⁰ is a hydrogen atom, a halogen atom, a C₁₋₆ alkyl group or a C₂₋₆ alkynyl group, and P¹ and P² are the same as or different from each other and are hydrogen atoms or
protecting groups for a hydroxy group, 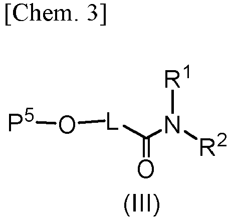
wherein R¹ and R² are the same as or different from each other and are hydrogen atoms, substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, P⁵ is a hydrogen atom or a protecting group for a hydroxy group, and L is a substitutable C₁₋₆ alkylene group.

2. The production method according to claim 1, wherein the boron reagent is a boron trifluoride diethyl ether complex.

3. The production method according to claim 1, wherein 1 molar equivalent of the compound of formula (II) is reacted with the compound of formula (III) in the presence of 1.0 molar equivalent to 10 molar equivalents of the boron reagent.

4. The production method according to claim 1, wherein P⁵ is a silyl-based protecting group.

5. The production method according to claim 4, wherein P⁵ is a trimethylsilyl group.

6. The production method according to claim 1, wherein L is a 1,2-ethylene group.

7. The production method according to claim 1, wherein R¹ and R² are the same as or different from each other and are hydrogen atoms or C₁₋₆ alkyl groups.

8. The production method according to claim 7, wherein R¹ is a hydrogen atom and R² is a C₁₋₃ alkyl group.

9. The production method according to claim 1, wherein R¹⁰ is a hydrogen atom or a methyl group.

10. The production method according to claim 1, wherein the solvent is selected from ether solvents or amide solvents.

11. The production method according to claim 1, wherein P¹ is a silyl-based protecting group and P² is a hydrogen atom.

12. The production method according to claim 11, wherein P¹ is a t-butyldiphenylsilyl group.

13. The production method according to claim 11, wherein the solvent is selected from ether solvents.

14. The production method according to claim 13, wherein the solvent is at least one selected from the group consisting of 1,2-dimethoxyethane, tetrahydrofuran, 4-methyltetrahydropyran and dioxane.

15. The production method according to claim 11, further comprising a step (iii) of deprotecting the silyl-based protecting group of P¹ to generate a 5'-hydroxy group, after the 2' substitution reaction step (ii).

16. The production method according to any one of claims 1 to 10, wherein P¹ and P² are hydrogen atoms.

17. The production method according to claim 16, wherein the solvent is selected from amide solvents.

18. The production method according to claim 17, wherein the solvent is N,N-dimethylacetamide.

19. A method for producing a uridine amidite compound of the following formula (V), the method comprising:
producing a compound of the following formula (II-2) by using the production method according to any one of claims 1 to 18; 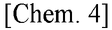 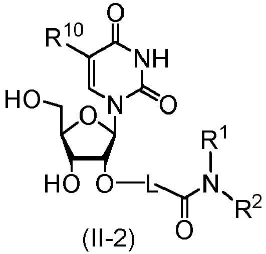
wherein R¹, R², R¹⁰ and L are as described above, and
then a step (iv) of converting a 5'-hydroxy group of the compound of formula (II-2) into a protecting group for a hydroxy group; and
a step (v) of converting a 3'-hydroxy group into an amidite group of the following formula (PA) with an amidite-forming reagent, 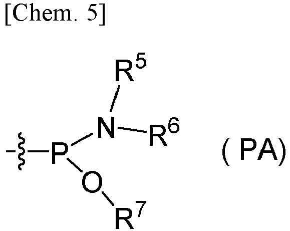
wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group, 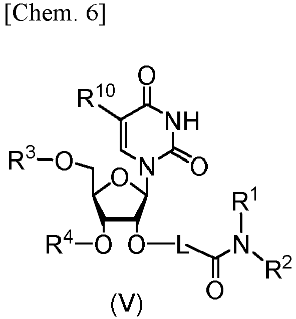
wherein R¹, R², R¹⁰ and L are as described above,
R³ is a protecting group for a hydroxy group, and R⁴ is an amidite group of formula (PA).

20. A method for producing a cytosine compound of the following formula (B), the method comprising:
a step (b) of reacting, in a solvent,
a protected uridine compound of the following formula (A) with a sulfonyl halide compound, and then with ammonia water, 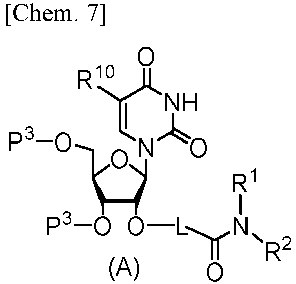
wherein R¹ and R² are the same as or different from each other and are hydrogen atoms,
substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, Lisa substitutable C₁₋₆ alkylene group, and P³ are the same as or different from each other and are substitutable benzoyl groups. 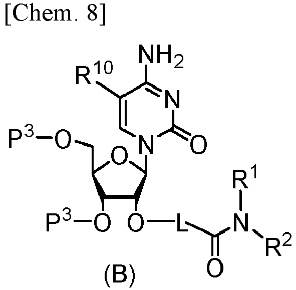
wherein R¹ and R² are the same as or different from each other and are hydrogen atoms,
substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkenyl group, L is a substitutable C₁₋₆ alkylene group, and P³ are the same as or different from each other and are substitutable benzoyl groups.

21. The production method according to claim 20,
wherein the sulfonyl halide compound is of the following formula (C): 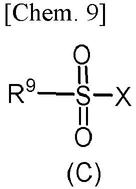
wherein X is a halogen atom, and R⁹ is a substitutable C₁₋₆ alkyl group or a substitutable C₆₋₁₄ aryl group.

22. The production method according to claim 21, wherein the sulfonyl halide compound is at least one selected from the group consisting of methanesulfonyl chloride, p-toluenesulfonyl chloride, and 2,4,6-triisopropylbenzenesulfonyl chloride.

23. The production method according to claim 20, comprising,
before the step (b),
a step (a1) of reacting a uridine compound of the following formula (II-2) with a benzoylation reagent to synthesize the protected uridine compound of formula (A), and
a step (a2) of obtaining the protected uridine compound of formula (A) by solid-liquid separation, 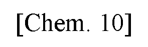 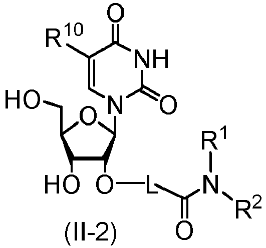
wherein R¹ and R² are the same as or different from each other and are hydrogen atoms,
substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, and Lisa substitutable C₁₋₆ alkylene group.

24. A method for producing a cytosine amidite compound of the following formula (G), comprising
producing a cytosine compound of formula (B) by the production method according to any one of claims 20 to 23, and using the cytosine compound as a starting material, 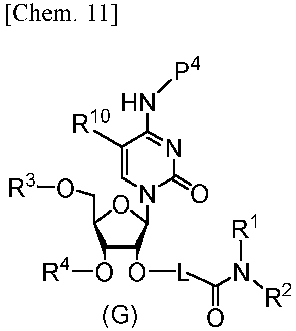
wherein R¹ and R² are the same as or different from each other and are hydrogen atoms,
substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, P⁴ is a protecting group for an amino group, R³ is a protecting group for a hydroxy group, R⁴ is a cytosine amidite compound of the following formula (PA), wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group,

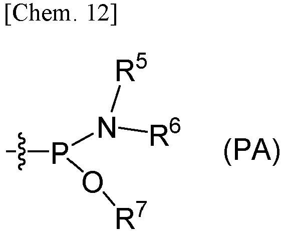

25. The production method according to claim 24, comprising:
a step (c) of reacting the cytosine compound of formula (B) with a primary or secondary alkylamine to obtain a cytosine compound of the following formula (D): 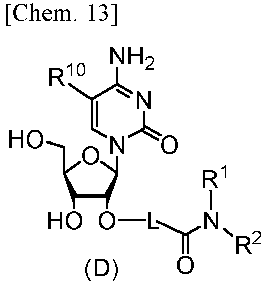
wherein R¹ and R² are the same as or different from each other and are hydrogen atoms,
substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, and Lisa substitutable C₁₋₆ alkylene group.

26. The production method according to claim 25, further comprising:
a step (d1) of converting the 5'-hydroxy group in the cytosine compound of formula (D) into a protecting group for a hydroxy group; and
a step (d2) of protecting an amino group of a cytosine base to produce a cytosine compound of the following formula (F): 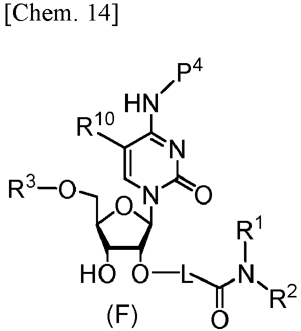
wherein R¹ and R² are the same as or different from each other and are hydrogen atoms,
substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, P⁴ is a protecting group for an amino group, and R³ is a protecting group for a hydroxy group.

27. The production method according to claim 26, further comprising:
a step (e) of converting the cytosine compound of formula (F) into a cytosine amidite compound of the following formula (G) with an amidite-forming reagent, 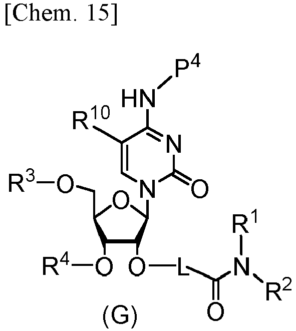
wherein R¹ and R² are the same as or different from each other and are hydrogen atoms,
substitutable C₁₋₆ alkyl groups, substitutable C₇₋₁₆ aralkyl groups, substitutable C₂₋₆ alkenyl groups, substitutable C₂₋₆ alkynyl groups or substitutable C₆₋₁₄ aryl groups, R¹⁰ is a hydrogen atom, a halogen atom, a C₁₋₆ alkyl group or a C₂₋₆ alkynyl group, L is a substitutable C₁₋₆ alkylene group, P⁴ is a protecting group for an amino group, R³ is a protecting group for a hydroxy group, R⁴ is a cytosine amidite compound of the following formula (PA), wherein R⁵ and R⁶ are the same as or different from each other and are C₁₋₆ alkyl groups, or R⁵ and R⁶ are bonded to each other to form a ring together with the nitrogen atom to which they are bonded, and R⁷ is a protecting group for a phosphate group,

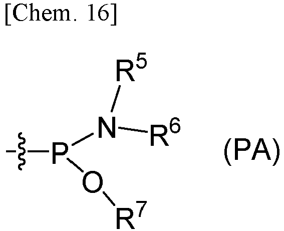

28. The production method according to claim 27, wherein R⁵ and R⁶ are i-propyl groups, and R⁷ is a 2-cyanoethyl group.

29. The production method according to claim 24, wherein R³ is a 4,4'-dimethoxytrityl group.

30. The production method according to claim 24, wherein P⁴ is an acetyl group, a propionyl group, an i-butyryl group, a pivaloyl group, a n-butyryl group, a benzoyl group or a phenoxyacetyl group.
